# EUROPEAN PATENT SPECIFICATION

(11) **EP 1 718 743 B1**
(45) Date of publication and mention of the grant of the patent: **26.08.2009**
(21) Application number: 05712812.6
(22) Date of filing: 04.02.2005
(51) Int. Cl.: C12N 9/96, C12N 9/00, C12Q 1/68, C12P 19/34, G01N 33/53

(54) **ANTI-FREEZE PROTEIN ENHANCED NUCLEIC ACID AMPLIFICATION**
DURCH GEFRIERSCHUTZPROTEIN VERBESSERTE NUKLEINSÄUREAMPLIFIKATION
AMPLIFICATION D'ACIDES NUCLEIQUES AMELIOREE PAR PROTEINES ANTIGEL

(30) Priority: 04.02.2004 US 541999 P
(43) Date of publication of application: 08.11.2006
(73) Proprietor: QIAGEN North American Holdings, Inc., Germantown, MD 20874 (US)
(72) Inventor: WESTBERRY, Ryan, Smith, Westminster, CO 80234 (US); PETERS, Lars-Erik, Lafayette, CO 80026 (US); GREENLEE, Jessica, Jaclyn, Lafayette, CO 80026 (US)
(74) Representative: Schneider, Jürgen M.
(86) International application number: PCT/US2005/003502
(87) International publication number: WO 2005/076908

(56) References cited:
- WO-A1-92/12722
- VAJDA T ET AL: 'Cryo-biorganic chemistry: molecular interactions at low temperatures.' CMLS. CELL MOL LIFE SCIENCE. vol. 56, 1999, pages 398 - 414, XP008072167
- UPRETI ET AL: 'Enzyme leakage during cryopreservation of ram spematoza.' ANIMAL REPRODUCTION SCIENCE. vol. 41, 1996, pages 27 - 36, XP008072166

## Description

### FIELD OF THE INVENTION

The invention generally relates to methods and compositions for increasing amplification yields in nucleic acid amplification reactions, for enhancing signal intensity and improving the signal to noise ratio in nucleic acid detection and quantification methods, such as real time polymerase chain reactions (real time PCR), and for increasing the stability of amplification enzyme solutions for use in such reactions over repeated freeze/thaw cycles.

### BACKGROUND OF THE INVENTION

The ability to prepare large amounts of nucleic acid molecules is requisite to a number of protocols in molecular biology, as well as a basic requirement in numerous downstream uses in biotechnology and clinical research. For example, amplified nucleic acid molecules are often used in cloning experiments, DNA sequencing reactions, restriction digestion reactions, and subsequent ligation reactions, and these uses are all, or to some extent, dependent on the quality and quantity of the starting DNA material. As such, there has been, and continues to be, a need for reliable methods for preparing large amounts of quality, sequence-specific nucleic acid molecules.

In addition, the ability to detect and/or quantify target nucleic acid molecules from a mixed starting material is useful in a number of clinical, industrial and basic research applications. For example, sensitive and accurate detection and quantification of viral nucleic acid sequences in a patient sample is helpful in a clinical setting for accurate diagnosis and subsequent treatment of a patient. Such detection and quantification processes generally require amplification of one or more target nucleic acid molecules present in the starting material. As such, there has been, and continues to be, a need for facilitating the detection and quantification of target nucleic acid sequences from a starting material, which again requires reliable methods for preparing large amounts of quality, sequence-specific nucleic acid molecules.

The predominant approach for amplifying nucleic acid is via the polymerase chain reaction (PCR). PCR is a convenient *in vitro* amplification process useful in the exponential increase of template nucleic acid. Particular applications of PCR include the detection and/or quantification of target gene expression as well as confirmation of differential expression of target genes detected using array techniques. In general, optimization of PCR techniques could facilitate both the accuracy (i.e. sequence specificity) and total levels or amounts of the amplified product (amplicon), and optimization of real time-PCR techniques could facilitate the sensitivity and suppress nonspecific amplification during the procedure. Each real time assay relies upon the release of a detectable signal upon production of a PCR product, and nonspecific amplification is particularly problematic when the starting material is small. In addition, there is a need for greater stability of the constituent enzymes involved in the PCR reaction, both during the reaction and during storage of the relevant constituents of the reaction prior to combination with the reaction mixture.

Conventionally PCR optimization has focused on modifying standard PCR buffers, altering primer annealing temperatures, providing more effective thermostable polymerase enzymes, and designing more effective primer molecules. With regard to product quantification in real-time PCR, optimization has focused on the development of two relatively new assays: the TaqMan^{™} method of real time PCR employing intercalating dyes and binary hybridization probes (Lee et al., 1993 Nucleic Acids Res., 21 (16:3761.6)). In either situation, further reduction of non-specific amplification, enhancement of signal intensity and facilitation of enzyme stability is critically needed to provide more sensitive, accurate and robust results.

Against this backdrop the present invention has been developed.

### SUMMARY OF THE RELEVANT LITERATURE

Anti-freeze proteins (AFPs) are a class of ice-binding proteins prevalent in organisms that live at or below freezing temperatures. The first AFP discovered was from the blood of a species of Antarctic fish. DeVries (1969) Science 163, 1073-1075. Since these early discoveries, a number of organism have been identified that express these proteins, including fish, insects, plants and microorganisms. Jia et al. (2002) TRENDS in Biochem. Sciences, 27:2, 101-106. AFPs have been classified based on protein sequence and structural characteristics, a number of which have significantly different structures. A number of different AFP-based structures have been identified within these analysis, including: helix bundle proteins, helical proteins with Thr residues arrayed on one side of the protein, helical proteins with Ala-Ala-Thr repeats, and globular type proteins.

In recent years, AFP proteins, and especially AFP type I proteins, have been used in cosmetic applications for general product stability, in addition, AFP has been used in a limited setting as a food additive to help protect frozen food under freezing conditions, for cold protection in mammalian cells, and for enhanced tumor cell destruction during cytosurgery. Fletcher et al. (1999) CHEMTECH 30(6):17-28.

Spermatozoal motility and enzyme leakage during cryopreservation of ram spermatozoa were also evaluated in the presence and absence of anti-freeze proteins. Upseti et al. (1996) Animal Reproduction Science 41, 27-36

### SUMMARY OF THE INVENTION

The present invention provides compositions and methods for improving the performance of nucleic acid amplification reactions, where the reaction typically comprises at least one cycle of a denaturation step, an annealing step, and an extension step. Compositions of the present invention provide the beneficial effect of improving enzyme stability over the course of numerous freeze/thaw events and, more surprisingly, also significantly enhance the performance of the amplification reaction and dramatically increased signal intensity and improve signal to noise ratios in associated detection and quantification methods. The present invention provides amplification reaction mixture . compositions comprising an anti-freeze protein (AFP) and an enzyme, wherein said enzyme is a polymerase, an AFP combined with a carrier protein such as, *e.g.,* BSA and the like and an enzyme, wherein said enzyme is a polymerase , an AFP combined with a polyol, and an AFP combined with a carrier protein and a polyol and an enzyme, wherein said enzyme is a polymerase.

In one aspect of the present invention, methods and compositions are provided for improving the stability of an enzyme in an amplification enzyme solution, where the enzyme solution is subjected to at least one freeze/thaw cycle before use in a subsequent amplification reaction. In one embodiment, the enzyme solution comprises AFP, and optionally further comprises a carrier protein and/or a polyol. In a preferred embodiment, the AFP is added to the enzyme solution prior to, or contemporaneous with, the freeze/thaw cycle.

In another aspect of the present invention, methods and compositions are provided for improving the performance of nucleic acid amplification reactions in general, and related detection and quantification methods in particular, comprising including in the amplification reaction mixture at least one anti-freeze protein optionally in combination with a carrier protein in a zwitterionic buffer. As demonstrated herein, these novel amplification reaction mixtures improve amplicon yield and signal intensity, increase the signal-to-noise ratio, and enhance the overall sensitivity of the reactions.

In a particular embodiment, improved nucleic acid detection and quantification methods are provided comprising a nucleic acid amplification reaction in which the reaction mixture comprises at least one anti-freeze protein to improve the signal-to-noise ratio of the reaction. Preferred anti-freeze protein(s) have one or more alanine-rich motifs for enhancement of signal and sensitivity. In particularly preferred embodiments, a carrier protein such as, *e.g.,* BSA or other like protein, is Included with the anti-freeze protein in the reaction mixture to maximize the signal-to-noise ratio. Surprisingly, the combination of AFP and carrier protein provides a synergistic improvement in the signal-to-noise ratio over AFP alone or carrier protein alone.

In a preferred embodiment, a polyol is included in amplification reaction mixtures comprising a template nucleic acid having a degree of secondary structure. In a particularly preferred embodiment, the polyol is selected from the group consisting of sorbitol, maltitol, adonitol, arabitol and mannitol. Ancillary materials can also be included with these reaction mixtures, including, but not limited to single-stranded binding protein, n-propyl sulfoxide, and the like, to further facilitate the amplification reaction.

Additional embodiments of the present invention include compositions for maximizing signal amplification during real time PCR where release of fluorescent signal upon 5'-3' nucleolytic degradation is anticipated. Preferred compositions and methods of the present invention employ reaction mixtures comprising at least one anti-freeze protein, preferably having one or more alanine rich motifs (for example exhibited by AFP type I), alone or in combination with a carrier protein. In particularly preferred embodiments, the reaction mixtures are prepared with a zwitterionic buffer having a pH of between about 7.9 and about 8.1. In another preferred embodiment, the nucleic acid quantification method is real time PCR and the addition of the anti-freeze protein and carrier protein provides for synergistic signal amplification.

These and various other features and advantages of the invention will be apparent from a reading of the following detailed description and a review of the appended claims.

### BRIEF DESCRIPTION OF THE DRAWINGS

Figure 1 is a stained 1 % agarose gel showing yields of PCR reaction products from reactions having from 0 to 200 mg/ml anti-freeze protein 1 included within the reaction.
Figures 2A and 2B graphically illustrate signal amplification synergy between anti-freeze protein 1 and BSA during PCR (A) Average RFU and (B) Threshold Cycle (Ct).
Figures 3A and 3B graphically illustrate that inclusion of BSA and AFP1 in the PCR Master Mix facilitates storage of the buffer at -20°C. Figure 3A shows average RFU values and Figure 3B shows threshold cycle values. Buffer samples were either: BSA alone, AFP1 alone, BSA and AFP1 combined or no additives.
Figures 4A and 4B graphically illustrate PCR buffer compositions including AFP1, BSA, and assorted combinations of buffer and salt, (A) threshold cycle and (B) average RFU.
Figure 5 graphically compares the threshold cycle for Real-Time PCR products prepared in 25mM TAPS-KOH with 15mM KCI pH8, 25mM TAPS-Tris with 50mM KCI pH 8.0, and 25mM Bicine-Tris with 50mM KCI pH 8.4.
Figure 6 graphically compares the threshold cycle for Real-Time PCR products prepared in 25mM Bicine-Tris with 50mM KCI pH 8.4, 25mM TAPS-Tris with 50mM KCI pH 8.0, and Invitrogen Platinum qPCR Supermix-UDG, used according to manufacturers standards.
Figure 7 graphically compares the threshold cycle for real-time PCR products prepared in the presence of no additives, BSA, AFGP, AFGP and BSA, AFP1 and BSA, and AFGP, AFP1 and BSA.
Figures 8A and 8B graphically compares the RFU for long term stability testing of an embodiment of the present invention as compared to testing performed with the Invitrogen Platinum qPCR Supermix UDG product, purchased and performed using Invitrogen protocol.
Figures 9A and 9B graphically represent that inclusion of 100mM sorbitol in RT-PCR Master Mix facilitates storage stability at -20°C. Figure 9A shows Ct values and Figure 9B shows RFU values from PCR performed after freeze/thaw on sorbitol containing and non-containing master mix buffers for one or three weeks. Samples were either stored at 4, -20 or -80°C.
Figures 10A and 10B are stained 1% agarose gels showing RT PCR reaction products after one-week (10A) or three-week (10B) of storage at either 4, -20 or -80°C in zero, 100mM, or 200mM sorbitol.
Figure 11 graphically represents the performance consistency of the embodiment of the present invention across 15 freeze/ thaw cycles.

### DETAILED DESCRIPTION OF THE INVENTION

The following definitions are provided to facilitate understanding of certain terms used frequently herein and are not meant to limit the scope of the present disclosure:

As used herein, "amplification reaction" or "nucleic acid amplification reaction" refers to any *in vitro* method for increasing the number of copies of a desired nucleic acid sequence with the use of a DNA polymerase. Nucleic acid amplification reactions include, for example, the polymerase chain reaction (PCR) (as described in U.S. Patent Nos. 4,683,195 and 4,683,202, ), Nucleic Acid Sequence-Based Amplification (NASBA) (as described in U.S Patent No. 5,409,818,) and Strand Displacement Amplification (SDA) (as described in U.S. Patent No. 5,455,166, ). As is well known in the art, such reactions find advantageous use in numerous nucleic acid detection methods for determining the presence of one or more target nucleic acid sequences in a sample, as well as in a wide variety of nucleic acid quantification methods for quantifying the amount of amplicon(s) produced by the reaction.

As used herein, "antisense" refers to polynucleotide sequences that are complementary to target "sense" polynucleotide sequences.

As used herein, "carrier protein(s)" refers to Bovine Serum Albumin (BSA), Prionex, , Cold Water Fish Gelatin, gelatin, Gro L, Gro S, DNAK, Heat Shock Protein 70 (HSP70), Apolipoprotein, as well as other like serum albumins.

As used herein, "Ct shift" or "threshold cycle" refers to the cycle at which an amplification product is detectable, a Ct shift of 1.5 to 3 cycles is equivalent to an approximate 5 to 10 fold higher DNA.

As used herein, "nucleic acid" or "NA" refers to both a deoxyribonucleic acid (DNA) and a ribonucleic acid (RNA), as well as modified and/or functionalized versions thereof. Similarly, the term "nucleotide" as used herein includes both individual units of ribonucleic acid and deoxyribonucleic acid as well as nucleoside and nucleotide analogs, and modified nucleotides such as labeled nucleotides. In addition, "nucleotide" includes non-naturally occurring analog structures, such as those in which the sugar, phosphate, and/or base units are absent or replaced by other chemical structures. Thus, the term "nucleotide" encompasses individual peptide nucleic acid (PNA) (Nielsen et al., Bioconjug. Chem. 1994; 5(1):3-7) and locked nucleic acid (LNA) (Braasch and Corey, Chem. Biol. 2001; 8(1):1-7) units.

As used herein, "polynucleotide," "oligonucleotide" or grammatical equivalents thereof means at least two nucleotides covalently linked together. As will be appreciated by those of skill in the art, various modifications of the sugar-phosphate backbone may be done to increase the stability of such molecules in physiological environments, including chemical modification such as, e.g., phosphorothioate or methyl phosphonate. Further, such molecules may be functionalized by coupling with one or more molecules having distinct characteristic properties for purposes of, e.g., facilitating the addition of labels.

As used herein, "nucleic acid sequence" refers to the order or sequence of nucleotides along a strand of nucleic acids. In some cases, the order of these nucleotides may determine the order of the amino acids along a corresponding polypeptide chain. The nucleic acid sequence thus codes for the amino acid sequence. The nucleic acid sequence may be single-stranded or double-stranded, as specified, or contain portions of both double-stranded and single-stranded sequences. The nucleic acid sequence may be composed of DNA, both genomic and cDNA, RNA, or a hybrid, where the sequence comprises any combination of deoxyribo- and ribo-nucleotides, and any combination of bases, including uracil (U), adenine (A), thymine (T), cytosine (C), guanine (G), inosine, xathanine hypoxathanine, isocytosine, isoguanine, etc.

As used herein, "complementary" or "complementarity" refers to the ability of a nucleotide in a polynucleotide molecule to form a base pair with another nucleotide in a second polynucleotide molecule. For example, the sequence 5'-A-C-T-3' is complementary to the sequence 3'-T-G-A-5'. Complementarity may be partial, in which only some of the nucleotides match according to base pairing, or complete, where all the nucleotides match according to base pairing. For purposes of the present invention "substantially complementary" refers to 95% or greater identity over the length of the target base pair region.

As used herein, "expression" refers to transcription and translation occurring within a host cell. The level of expression of a DNA molecule in a host cell may be determined on the basis of either the amount of corresponding mRNA that is present within the cell or the amount of DNA molecule encoded protein produced by the host cells. Further detail for the term "expression" within the context of the present invention can be obtained via a review of Sambrook et al., 1989, Molecular Cloning; A Laboratory Manual; 18.1-18.88.

As used herein, "freeze/thaw" conditions are characterized by freezing a target material at a temperature typically below 0°C and preferably at a temperature not to go below about -30°C to about -40°C. Thaw conditions typically do not exceed room temperature. A typical slow-freeze/thaw cycle is where a material is frozen between about 0°C and about -40°C, stored at that temperature for some period of time, and thawed between about 1°C to about 27°C, dependent on the end use required.

As used herein, "host cell" or "host cells" refers to cells expressing or capable of expressing a heterologous polynucleotide molecule, for example a plasmid vector. Host cells of the present invention express polynucleotides encoding polypeptides useful in any number of uses, including biotechnological, molecular biological and clinical settings. Examples of suitable host cells in the present invention include, but are not limited to, bacterial, yeast, insect and mammalian cells. Specific examples of such cells include, E. Coli DH5a cells, as well as various other bacterial cell sources, for example the E. Coli strains: DH10b cells, XL1Blue cells, XL2Blue cells, Top10 cells, HB101 cells, and DH12S cells, and yeast host cells from the genera including Saccharomyces, Pichia, and Kluveromyces.

As used herein, "isolated" and "purified" for purposes of the present invention are interchangeable, land refer to a polynucleotide, for example a target nucleic acid sequence, that has been separated from cellular debris, for example, high molecular weight DNA, RNA and protein. This would include an isolated RNA sample that would be separated from cellular debris, including DNA.

As used herein, "protein," "peptide," and "polypeptide" are used interchangeably to denote an amino acid polymer or a set of two or more interacting or bound amino acid polymers.

As used herein, "real-time PCR" refers to quantitative PCR techniques that typically use fluorescence probes, beacons, and/or intercalating dyes during all cycles of the process.

As used herein, "stringency" refers to the conditions, i.e., temperature, ionic strength, solvents, and the like, under which hybridization between polynucleotides occurs. Hybridization being the process that occurs between the primer and template DNA during the annealing step of the amplification process.

Embodiments of the present invention provide methods and compositions for enhancing the overall performance of nucleic acid amplification reactions and improving related detection and quantification methods. In particular, embodiments of the present invention are directed toward facilitating the signal intensity of nucleic acid amplification reactions in general, enhancing signal amplification and/or improving signal-to-noise ratios in nucleic acid detection and quantification methods, and increasing the stability for enzyme solutions through one or several freeze/thaw cycles before their use in such reactions and methods.

Preferably, specific embodiments of the present invention include the following: inclusion of anti-freeze protein(s) (AFP) to enhance both signal amplification (RFU) and sensitivity (threshold cycle) during real-time PCR; inclusion of AFP within enzyme solutions to enhance storage stability during freeze/thaw conditions; inclusion of a carrier protein, for example BSA, with the AFP to provide an additional synergistic effect on signal size and sensitivity, as well as on storage stability; inclusion of sorbitol or other like polyol material with the AFP in a mixture to both enhance the product yield and further improve stability during freeze/thaw cycles; and inclusion of dUTP, polyol, and AFP in a zwitterionic buffer formulation, i.e., TAPS-Tris with KCL or TAPS-KOH with KCI based buffer, to provide a high performance nucleic acid amplification buffer, e.g., PCR buffer (increases sensitivity, specificity, signal intensity and storage stability), especially where the pH is between about 7.9 and about 8.1.

### Anti-Freeze Proteins (AFP)

Anti-freeze proteins (AFPs) represent a family of proteins that contribute freeze resistance and freeze tolerance to a number of species that thrive in freezing conditions. In general, AFP molecules within the organism bind to the surface of seed ice crystals and control the crystal's growth. See Jia et al., (2002) TRENDS in Biochem. Sciences, 27(2) 101-106. A number of different organisms have been found to express AFPs, including several species of fish and several species of insect. Fish AFPs are classified into five groups (see Table 1), based principally on primary and secondary structural analysis. With regard to insect AFPs, several insect species, including Tm or Dc (beetle) and Cf (moth), have been classified into two groups. AFPs have been isolated from several plant species as well Atici et al., (2003) Phytochemistry, 64(7) 187-96.

Importantly, several different identified structural motifs and motif repeats within the AFP family have been shown to function similarly to inhibit ice growth, including an alanine-rich helix having 11 amino acids (aa) (three turns of a helix) (AFP Type 1) or a 3 aa repeat of Ala-Ala-X (side-chain disaccharide) motif (AFGP) where X is serine, arginine, lysine, asparagine, glutamine, or threonine. Additional structural information can be obtained from Jia et al. and is shown in Table 1 (see also U.S. Patent No. 6,017,574 ).

**Table 1: Structural Characteristics of AFPs**

| Characteristic | AFGP (Anti-Freeze Glycoprotein) | AFP Type I | AFP Type II | AFP Type III | AFP Type IV |
|---|---|---|---|---|---|
| MW | ~2,600-33,000 | ~3,300-4,500 | ~11,000-24,000 | ~6,500 | ~12,200 |
| 1° Structure | (ala-ala-thr)n disaccharide | ala rich repeats - typically 11 aa repeats | cystine rich disulphide linked | general | 17% gluramine; lack of disulphide bridges |
| Carbohydrate Linked | Yes | No | Generally not | No | No |
| 2° Structure | Expanded | alpha helical amphiphilic | beta sheet | beta sandwich | amphipathic alpha helix |
| 3° Structure | NA | 100% helix | NA | NA | Four-helix Antiparallel Bundle |
| Biosynthesis | Multi-protein | Prepro AFP | Prepro-AFP | Pro-AFP | No Post-translational Modifications |
| Protein Components | 8 | 7 | 2-6 | 12 | 1 |
| Gene Copies | NA | 80-100 | 15 | 30-150 | NA |
| Natural Source | Antarctic Notothenioids; Northern Cod | Right-eyed Flounders; Sculpins | Sea raven; smelt; herring | Ocean pout; Wolffish | Longhorn Sculpin |

The present invention provides compositions and methods for enhancing signal amplification in nucleic acid detection and quantification methods such as, *e.g.,* real-time PCR, as well as for increasing the stability of enzyme solutions employed in nucleic acid amplification reactions.

In one embodiment, the improved amplification reaction mixtures provided herein generally include primer(s), template DNA, dNTPs and an appropriate amplification enzyme, for example Taq DNA polymerase, and specifically include at least one AFP. In preferred embodiments, the AFP is selected from the group having an alanine-rich helix or a repetitive repeat of -Ala-Ala-X-(disaccharide), derived from AFP type I or AFGP, respectively. In addition, fragments of AFP including the alanine-rich helix motif derived from AFP type I (See for example U.S. Patent No. 5,925,540, ), or repetitive repeats of -Ala-Ala-Thr-(disaccharide) derived from AFGP, and variants, derivatives, isoforms and fusion proteins thereof (all these examples are referred to generally as AFP for purposes of the invention) are useful in these regards. In preferred embodiments the amplification enzyme solution and/or reaction mixture further includes a carrier protein, for example BSA or gelatin, to enhance the AFP-derived results. As demonstrated herein, the inclusion of a carrier protein in the reaction mixture with AFP results in a synergistic effect greater than that of AFP alone or carrier protein alone.

Embodiments in accordance with the present invention typically include an enzyme solution of AFP from about 10-200 µg/ml, and preferably from 25-100 µg/ml and most preferably from about 40-60 µg/ml. Inclusion of carrier protein with the AFP is typically at a concentration of about 100 µg/ml to about 300 µg/ml, and preferably from about 150 to about 250 µg/ml, and most preferably about 200 µg/ml. Inclusion of AFP and carrier protein in standard real-time PCR buffers provide a significant improvement in signal amplification as measured by average RFU, and cycle of detection as measured by threshold cycle. As shown in the Examples that follow, the combination of AFP with a carrier protein provides a synergistic improvement in signal strength or intensity and sensitivity of such assays.

AFP for use in the present invention can be purchased from AFP Proteins, Inc., Waltham, MA. In addition, AFP, and fragments, derivatives and fusion proteins of AFP in accordance with the invention can be expressed in insect, yeast, prokaryote, and eukaryote cells. Suitable prokaryotic hosts to be used for expression include but are not limited to bacteria of the genera *Escherichia, Bacillus* and *Salmonella.* AFP sequence for design of recombinant protein expression can be obtained from a review of Jia *et al* (also see U.S. Patent No. 5,925,540).

Modifications of the amino acid sequence of AFP molecules useful in the present invention can be accomplished by a number of known techniques. For example, mutations may be introduced at particular locations by oligonucleotide-directed mutagenesis (Walder et al., 1986, Gene, 42:133; Bauer et al., 1985, Gene 37:73, Craik, 1985, Biotechniques, 12-19; Smith et al., 1981, Genetic Engineering: Principles and Methods, Plenum Press; U.S. Patent No. 4,737,462). Modifications may be useful in the enhancement of AFP activity as discussed herein and shown in the Examples.

AFP polypeptides of the present invention are preferably used in an isolated or partially isolated form. The polypeptides may be recovered and purified from recombinant cell cultures by known methods, including, for example, ammonium sulfate or ethanol precipitation, anion or cation exchange chromatography, phosphocellulose chromatography, hydrophobic interaction chromatography, affinity chromatography and lectin chromatography.

AFP polypeptides can be fused to heterologous polypeptides to facilitate purification. Many available heterologous peptides allow selective binding of the fusion protein to a binding partner. Non-limiting examples of peptide tags include 6-His, thioredoxin, hemagglutinin, GST, and the OmpA signal sequence tag. A binding partner that recognizes and binds to the heterologous peptide can be any molecule or compound, including metal ions, antibodies, antibody fragments, or any protein or peptide that preferentially binds the heterologous peptide to permit purification of the fusion protein.

The present invention further provides amplification enzyme solutions and amplification buffers, for example real-time PCR buffers, having enhanced stability for use after numerous freeze/thaw cycles, and extended storage times from 4°C to -80°C (see Examples below). As above, standard amplification buffer ingredients are mixed with AFP or with AFP and a carrier protein, using the concentrations discussed above, to minimize enzyme degradation between any two slow freeze/thaw cycles. Note that AFP is particularly effective in stabilizing against slow freeze events, for example freezing an enzyme solution between the temperatures of 0°C and -40°C.

As such, and without being bound by theory, it is believed that the inclusion of AFP or AFP in combination with a carrier protein results in the dual benefit of stabilizing the amplification enzyme during repetitive freeze/thaw cycles, and surprisingly further enhances the sensitivity and signal strength of the subsequent nucleic acid amplification reaction., Typically, stability is provided for freeze conditions between 0°C to about -40°C. Kits having an amplification buffer, for example a real-time PCR buffer, that include appropriate amounts of AFP can be packaged to maximize the sensitivity of the assay and stabilize the storage of the buffer under slow-freeze conditions. In addition, AFP or AFP and carrier protein can be packaged with a desired polymerase for addition to a PCR or other like amplification reaction mixture.

Note that although the inclusion of AFP in PCR buffers has been shown to enhance polymerase-based amplification reactions and detection and quantification methods employing the same, it is also envisioned to be useful in the storage of enzymes having other desirable activities. For example, AFP may find use in any enzyme solution that will undergo slow freezing conditions yet also requires the maintenance of enzyme activity, i.e., where the enzyme may be damaged by the freeze/thaw event. In one alternative embodiment, AFP, in the presence or absence of a carrier protein, is combined with AMV to protect the reverse transcriptase activity of the enzyme over a period of several slow freeze/thaw cycles.

### Nucleic Acid Amplification Using AFP-Based Reaction Mixtures

In one aspect, the invention provides methods for amplifying a nucleic acid template, comprising subjecting the nucleic acid template to an amplification reaction in an amplification reaction mixture. The amplification reaction mixture preferably comprises AFP1, and still more preferably comprises AFP1 and a carrier protein such as, *e.g.,* BSA.

Nucleic acid molecules may be amplified according to any of the literature-described manual or automated amplification methods. Nucleic acid amplification results in the incorporation of nucleotides into a DNA molecule or primer, thereby forming a new DNA molecule complementary to a nucleic acid template. The formed DNA molecule and its template can be used as templates to synthesize additional DNA molecules. As used herein, one amplification reaction may consist of many rounds or cycles of DNA replication. DNA amplification reactions include, for example, polymerase chain reactions ("PCR"). One PCR reaction may consist of 10 to 100 "cycles" of denaturation and synthesis of a DNA molecule. Such methods include, but are not limited to PCR (as described in U.S. Pat. Nos. 4,683,195 and 4,683,202, Strand Displacement Amplification ("SDA") (as described in U.S. Pat. No. 5,455,166, and Nucleic Acid Sequence-Based Amplification ("NASBA" (as described in U.S. Pat. No. 5,409,818). For example, amplification may be achieved by a rolling circle replication system which may even use a helicase for enhanced efficiency in DNA melting without heat (See Yuzhakou et al., "Replisome Assembly Reveals the Basis for Asymmetric Function in Leading and Lagging Strand Replication," Cell 86:877-886 (1996) and Mok et al., "The Escherichia coli Preprimosome and DnaB Helicase Can Form Replication Forks That Move at the Same Rate," J. Biol. Chem. 262:16558-16565 (1987),). Most preferably, nucleic acid molecules are amplified by the methods of the present invention using PCR-based amplification techniques.

In a preferred embodiment, the amplification reaction involves a high temperature denaturation step. Preferred temperatures for the high temperature denaturation step range from about 90°C to about 98°C, with temperatures from 93°C to 94°C being especially preferred. Such preferred amplification reactions include thermocycling amplification reactions, such as polymerase chain reactions involving from about 10 to 100 cycles, more preferably from about 25 to 50 cycles, and peak temperatures of from about 93°C to about 94°C.

In a preferred embodiment, a PCR reaction is done using a desired polymerase to produce, in exponential quantities relative to the number of reaction steps involved, at lease one specific nucleic acid sequence, given (a) that the ends of the requisite sequence are known in sufficient detail that oligonucleotiedes can be synthesized which will hybridize to them and (b) that a small amount of the sequence is available to initiate the chain reaction. The product of the chain reaction will be discrete nucleic acid duplex with termini corresponding to the ends of the specific primers employed.

Any source of nucleic acid, in purified or nonpurified form, can be utilized as the starting nucleic acid, provided it contains the specific nucleic acid sequence desired. Thus, the process may employ, for example, DNA or RNA, including messenger RNA, which DNA or RNA may be single stranded or double stranded. In addition, a DNA-RNA hybrid which contains one strand of each may be utilized. A mixture of any of these nucleic acids may also be employed, or the nucleic acids produced from a previous amplification reaction herein using the same or different primers may be so utilized. The nucleic acid amplified is preferably DNA. The specific nucleic acid sequence to be amplified may be only a fraction of a larger molecule or can be present initially as a discrete molecule, so that the specific sequence constitutes the entire nucleic acid. It is not necessary that the sequence to be amplified be present initially in a pure form; it may be a minor fraction of a complex mixture, such as a portion of the β-globin gene contained in whole human DNA or a portion of nucleic acid sequence due to a particular microorganism which organism might constitute only a very minor fraction of a particular biological sample. The starting nucleic acid may contain more than one desired specific nucleic acid sequence which may be the same or different. Therefore, the method is useful not only for producing large amounts of one specific nucleic acid sequence, but also for amplifying simultaneously more than one different specific nucleic acid sequence located on the same or different nucleic acid molecules.

The nucleic acid or acids may be obtained from any source and include plasmids and cloned DNA or RNA, as well as DNA or RNA from any source, including bacteria, yeast, viruses, and higher organisms such as plants or animals. DNA or RNA may be extracted from blood, tissue material such as corionic villi or amniotic cells by a variety of techniques such as that described by Maniatis et al., Molecular Cloning: A Laboratory Manual, (New York: Cold Spring Harbor Laboratory) pp 280-281 (1982).

Any specific nucleic acid sequence can be produced by the present methods. It is only necessary that a sufficient number of bases at both ends of the sequence be know in sufficient detail so that two oligonucleotide primers can be prepared which hybridize to different strands of the desired sequence and at relative positions along the sequence such that an extension product synthesized from one primer, when it is separated from its template (complement), can serve as a template for extension of the other primer into a nucleic acid of defined length. The greater the knowledge about the bases at both ends of the sequence, the greater the specificity of the primers for the target nucleic acid sequence, and, thus, the greater the efficiency of the process. It will be understood that the word primer as used hereinafter may refer to more than one primer, particularly in the case where there is some ambiguity in the formation regarding the terminal sequence(s) of the fragment to be amplified. For instance, in the case where a nucleic acid sequence is inferred from protein sequence information a collection of primers containing sequences representing all possible codon variations based on degeneracy of the genetic code can be used for each strand. One primer from this collection will be homologous with the end of the desired sequence to be amplified.

In some alternative embodiments, random primers, preferably hexamers, are used to amplify a template nucleic acid molecule. In such embodiments, the exact sequence amplified is not predetermined.

Oligonucleotide primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981),.
One method for synthesizing oligonucleotides on a modified solid support is described in U.S. Pat. No. 4,458,006. It is also possible to use a primer that has been isolated from a biological source (such as a restriction endonuclease digest).

The specific nucleic acid sequence is produced by using the nucleic acid containing that sequence as a template. If the nucleic acid contains two strands, it is necessary to separate the strands of the nucleic acid before it can be used as the template, either as a separate step or simultaneously with the synthesis of the primer extension products. This strand separation can be accomplished by any suitable denaturing method including physical, chemical, or enzymatic means. One physical method of separating the strands of the nucleic acid involves heating the nucleic acid until it is completely (>99%) denatured. Typical heat denaturation may involve temperatures ranging from about 80°C to 105°C for times ranging from 1 to 10 minutes. Strand separation may also be induced by an enzyme from the class of enzymes known as helicases or the enzyme RecA, which has helicase activity and is known to denature DNA. The reaction conditions suitable for separating the strands of nucleic acids with helicases are described by Cold Spring Harbor Symposia on Quantitative Biology, Vol. XLIII "DNA: Replication and Recombination" (New York: Cold Spring Harbor Laboratory, 1978), and techniques for using RecA are reviewed in C. Radding, Ann, Rev. Genetics, 16:405-37 (1982).

If the original nucleic acid containing the sequence to be amplified is single stranded, its complement is synthesized by adding one or two oligonucleotide primers thereto. If an appropriate single primer is added, a primer extension product is synthesized in the presence of the primer, an agent for polymerization, and the four nucleotides described below. The product will be partially complementary to the single-stranded nucleic acid and will hybridize with the nucleic acid strand to form a duplex of unequal length strands that may then be separated into single strands, as described above, to produce two single separated complementary strands. Alternatively, two appropriate primers may be added to the single-stranded nucleic acid and the reaction carried out.

If the original nucleic acid constitutes the sequence to be amplified, the primer extension product(s) produced will be completely complementary to the strands of the original nucleic acid and will hybridize therewith to form a duplex of equal length strands to be separated into single-stranded molecules.

When the complementary strands of the nucleic acid or acids are separated, whether the nucleic acid was originally double or single stranded, the strands are ready to be used as a template for the synthesis of additional nucleic acid strands. This synthesis can be performed using any suitable method. Preferably, a molar excess (for cloned nucleic acid, usually about 1000:1 primer:template, for the genomic nucleic acid, usually about 10⁶:1 primer:template) of the two oligonucleotide primers is added to the buffer containing the separated template strands. It is understood, however, that the amount of complementary strand may not be known if the process herein is used for diagnostic applications, so that the amount of primer relative to the amount of complementary strand cannot be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

Nucleoside triphosphates, preferably dATP, dCTP, dGTP, and dTTP are also added to the synthesis mixture in adequate amounts, and the resulting solution is preferably heated to a temperature from about 90°C-95°C for about 1 to 10 minutes, preferably from 15 sec to 2 minutes. After this heating period, the solution is allowed to cool to a temperature from about 60°C, which is preferable for the primer hybridization. The polymerase then performs nucleic acid synthesis at a temperature well above room temperature, preferably at a temperature from about 60 to 75°C.

The newly synthesized strand and its complementary nucleic acid strand form a double-stranded molecule which is used in the succeeding steps of the process. In the next step, the strands of the double-stranded molecule are separated using any of the procedures described above to provide single-stranded molecules.

New nucleic acid is synthesized on the single-stranded molecules. Additional polymerase, nucleotides, and primers may be added if necessary for the reaction to proceed under the conditions prescribed above. Again, the synthesis will be initiated at one end of the oligonucleotide primers and will proceed along the single strands of the template to produce additional nucleic acids.

The steps of strand separation and extension product synthesis can be repeated as often as needed to produce the desired quantity of the specific nucleic acid sequence. The amount of the specific nucleic acid sequence produced will increase in an exponential fashion.

When it is desired to produce more than one specific nucleic acid sequence from the first nucleic acid or mixture of nucleic acids, the appropriate number of different oligonucleotide primers are utilized. For example, if two different specific nucleic acid sequences are to be produced, four primers are utilized. Two of the primers are specific for one of the specific nucleic acid sequences and the other two primers are specific for the second specific nucleic acid sequence. In this matter, each of the two different specific sequences can be produced exponentially by the present process. Of course in instances where nucleic acid sequences are the same, primer sequences will be the same.

Additionally, as mentioned above, in an alternative embodiment, random primers, , are used to amplify a template nucleic acid molecule.

The present invention can be performed in a step-wise fashion where after each step new reagents are added, or simultaneously, wherein all reagents are added at the initial step, or partially step-wise and partially simultaneously, wherein fresh reagent is added after a given number of steps, for example adding new enzyme solution comprising AFP and optionally carrier protein. After the appropriate length of time has passed to produce the desired amount of the specific nucleic acid sequence, the reaction may be halted by inactivating the enzymes in any known manner or separating the components of the reaction.

Thus, in amplifying a nucleic acid molecule according to the present invention, the nucleic acid molecule is contacted with a composition comprising a polymerase in an appropriate buffer, preferably having AFP, and preferably AFP1, and most preferably having AFP and a carrier protein.

### Method For Enhancing Amplification Reactions After One Or More Freeze/Thaw Events

The present invention provides methods for maintaining the functional stability of a polymerase in a solution, preferably a polymerase in a PCR or real time-PCR buffer, although other polymerases like reverse transcriptase polymerase III, etc. are within the scope of the present invention. Initially, a polyol, AFP or AFP with carrier protein is combined with a desired amplification enzyme. The combination can occur in a standard amplification buffer or buffers consistent with more specific embodiments of the present invention. The combined enzyme-polyol, enzyme-AFP, or enzyme-AFP-carrier protein is then utilized in the reaction mixture for an amplification reaction. Remaining material, when frozen, is protected from some or all of the freeze/thaw effects. The enzyme solution thus obtained maintains the requisite enzymatic activity and can be used over the course of numerous freeze/thaw events, preferably as many as five or ten freeze/thaw events, more preferably as many as fifteen freeze/thaw events. In a preferred embodiment, the amplification reaction is a real time-PCR reaction. In another embodiment, the method incorporates an enzyme mixed with at least two of: a polyol, an AFP, and a carrier protein, and most preferably all three components are included. In an additional embodiment, an unconventional nucleotide is included in the amplification reaction mix to enhance the efficiency of the reactions by lowering the formation of primer-dimers.

In an alternative embodiment of the present invention, inclusion of one or more polyols in an amplification enzyme solution or buffer further increases stability under freeze/thaw conditions. In a preferred embodiment the amplification buffer is a standard PCR buffer. In one embodiment, sorbitol or other like polyol is included in a PCR buffer at a concentration of from about 50mM to about 500mM, preferably from about 50mM to about 400mM, and most preferably from about 100mM to about 300mM. As above, inclusion of other disruptive agents, i.e., DMSO, SSBP, and the like, with the polyol is anticipated to enhance the overall positive affect.

### Compositions Of High Performance Real Time PCR Buffers

The present invention further provides high performance amplification buffers for use in amplification reactions, preferably in standard PCR reactions and real-time PCR reactions. Buffers in accordance with the present invention can include anti-freeze protein (preferably AFP1, AFGP, mixtures of AFP1 and AFGP), carrier protein, dNTP mix, polyol, nucleic acid polymerase, preferably a thermophilic or hyperthermophilic polymerase, and have a modified pH, obtained through a buffer system that utilizes a zwitterionic formulation. Illustrative zwitterionic formulations include HEPES-KOH , TAPS-Tris, HEPES-Tris ,HEPES-KOH , TAPS-KOHor TAPS-Tris. Preferred embodiments of the present invention utilize a buffer system that includes TAPS-KOH and/or TAPS-Tris, and most preferably TAPS-Tris 0. Preferred pH ranges for these buffers are dependent on the final use, for example, buffers for use in real-time PCR are buffered to have a pH of from about 7.9 to about 8.7, and preferably from about 8.2 to about 8.7. Note that buffers for use in standard PCR are modified to have a pH of from about 7.9 to 8.9. In preferred embodiments, the potassium salt concentration his between 10mM to about 80mM. In preferred embodiments, the dNTP mix of the buffer system includes from about 10% to about 50% dUTP (in replacement of dTTPs in the dNTP mix), and more preferably from about 10% to about 40% dUTP (in replacement of dTTPs in the dNTP mix). Further, in some embodiments n-propyl sulfoxide, and/or trehalose can be included in the high performance buffer.

Concentrations of ingredients useful in embodiments of the high performance buffer are as shown in Table 2. Note that the preferred concentration for TAPS-KOH is 150mM KCI and for the TAPs-Tris is 500mM KCI, both with a final buffer pH of about 8.

**Table 2: High Performance PCR Buffer/Real-Time PCR Buffer**

| **Buffer Ingredient** | **Useful Concentration Range (Final)** | | **Preferred Concentration (Final)** | |
|---|---|---|---|---|
| Sorbitol, Trehalose, , maltitol and/or mixtures thereof | 10mM - 300mM | | 100mM | |
| dNTP Mix/% dUTP in Replacement of dTTP (or other dNTP analogs) | dATP | 100µM-500µM | dATP | 200µM |
| | dCTP | 100µM-500µM | dCTP | 200µM |
| | dGTP | 100µM-500µM | dGTP | 200µM |
| | dTTP+dUTP | 100µM-500µM | dTTP+dUTP | 200µM |
| | dTTP/dUTP | 75%:25% - 25%-75% | dTTP/dUTP | 75%:25% |
| Nucleic Acid Polymerase | 0.5 to 2 Units | | 2 Units | |
| Mg Ion Concentration | 3mM to 10mM | | 5mM and 8mM | |
| Potassium Salt Concentration | 10mM to 80mM | | 40mM to 60mM | |
| anti-freeze protein, e.g., AFP type I, AFGP or mixtures of same//Carrier Protein | 10µg/ml to 200µg/ml//100µg/ml to 300µg/ml | | 50µg/ml//100µg/ml | |
| Buffering Ingredient, e.g., TAPS-Tris | Taps | 10mM-40mM | Taps | 25mM |
| | Tris | 5mM-25mM | Tris | 10.3mM |

The present invention further provides the AFP zwitterionic buffer compositions discussed above having a monovalent potassium salt ranging in concentration from about 30mM to about 80mM, and preferably between about 40mM and 60mM. Additionally, magnesium ions in buffers of the present invention can be from about 2.5mM to about 10mM, and preferably from about 5mM and about 8mM.

It is also envisioned that embodiments using modified dNTP mixtures to limit primer-dimer formation in accordance with embodiments previously described can be included with embodiments of the high performance PCR buffers of the instant invention.
As such, embodiments using the zwitterionic buffer formulations can have one or more of AFP, carrier protein, sorbitol, mannitol, DMSO, SSBP, and a dNTP mix having a percentage of the dTTP or other dNTP replaced with an unconventional nucleotide like dUTP. Note also that the embodiments of the present invention can include a combination of AFP1 with DMSO (or other like compound) and a polyol, for example, sorbital (See for example, U.S. Patent No. 6,783,940,). Typically, the composition has a pH of between about 7.9 and 8.2 for optimal effects. Other pH can be used but with limited results.

### PCR and Real-Time PCR Buffer Kits

The present invention further provides kits that include the composition embodiments of the present invention. Kits can include PCR buffers of the invention, for example embodiments of the high performance PCR buffers of the invention, or alternatively, pre-determined stand alone amounts of AFP, sorbitol, mannitol, or other compositions of the invention, which are added to PCR buffers or are combined with amplification enzymes appropriate for the target use, combined with the invention. In addition, kit compositions can include combinations of AFP with different polymerase enzymes in the same or different tubes.

Having generally described the invention, the same will be more readily understood by reference to the following examples, which are provided by way of illustration and are not intended as limiting.

### EXAMPLES

### Example 1: Anti-Freeze Proteins (AFPs) Enhance Stability of Real Time-PCR Master Mix During Extended Periods of Storage at -20°

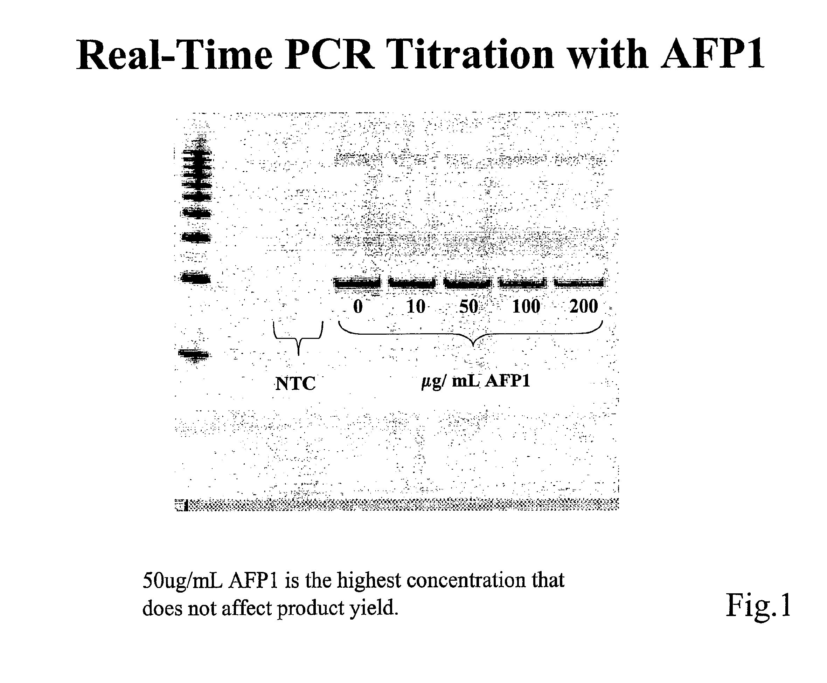

The effects of AFP1 on the freeze/thaw stability of real-time PCR buffers were investigated. Initially, AFP1 was titrated into real time-PCR reactions to determine what concentration of AFP1 did not detrimentally affect amplicon yield. Real-time PCR was performed as described in Eppendorf RealMasterMix Probe +/- ROX (catalog #00320900.525), and products visualized on ethidium bromide stained 1% agarose gels. As shown in Figure 1, product yields were not detrimentally affected until approximately 100 µg/ml AFP1 was included in the reaction mix. Conversely, the addition of 50 µg/ml AFP1 to the real time-PCR had little or no effect on the reaction yields (compare the lane having 0 AFP1 to the lane having 50 µg/ml AFP1).

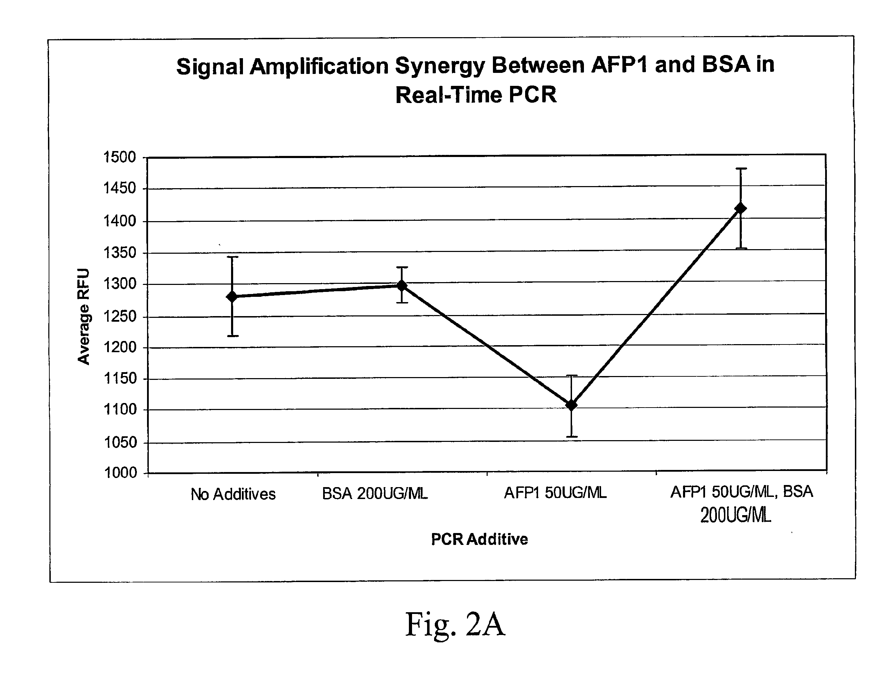

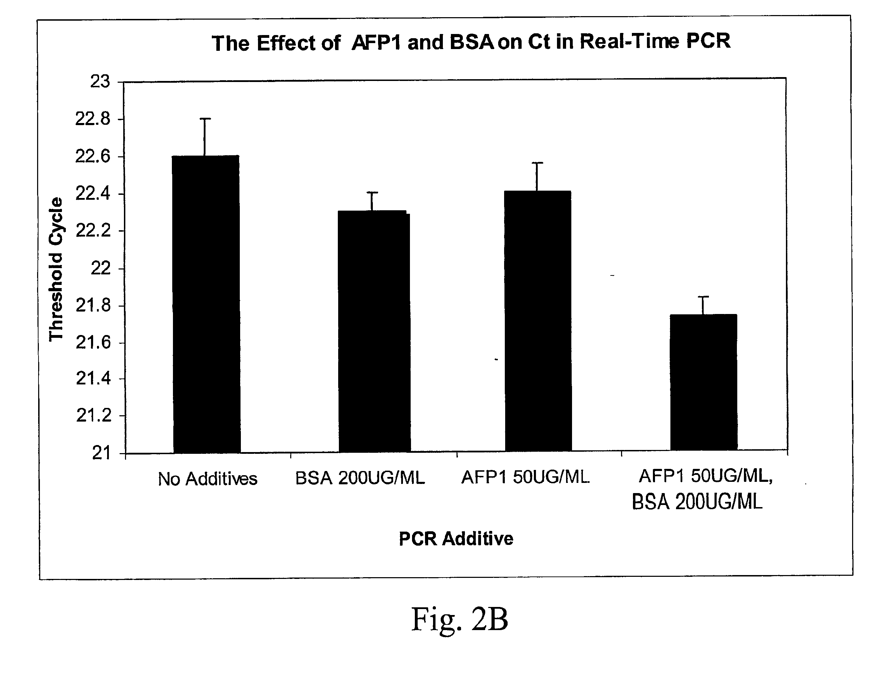

Prior to determining the freeze/thaw capacity of Real-Time PCR buffers in the presence and absence of AFP1, the ability of AFP1 to enhance fresh Real-Time PCR samples was tested. Real-Time PCR was performed as above and samples incubated with either 200 µg/ml BSA, 50 µg/ml AFP1, or 200 µg/ml BSA mixed with 50 µg/ml AFP1. Figures 2A and 2B graphically show that the addition of AFP1 alone actually has a slightly negative effect on the average RFU (A) and Threshold cycle (B) in this experiment. BSA alone had little or no effect on real-time PCR. However, the combination of BSA and AFP1 provided marked improvement on both signal amplification (A) and threshold cycle (B). As such, the combination of AFP1 and a carrier protein like BSA provides a synergistic benefit during real-time PCR while maintaining maximal yield of the amplicons (Figure 1).

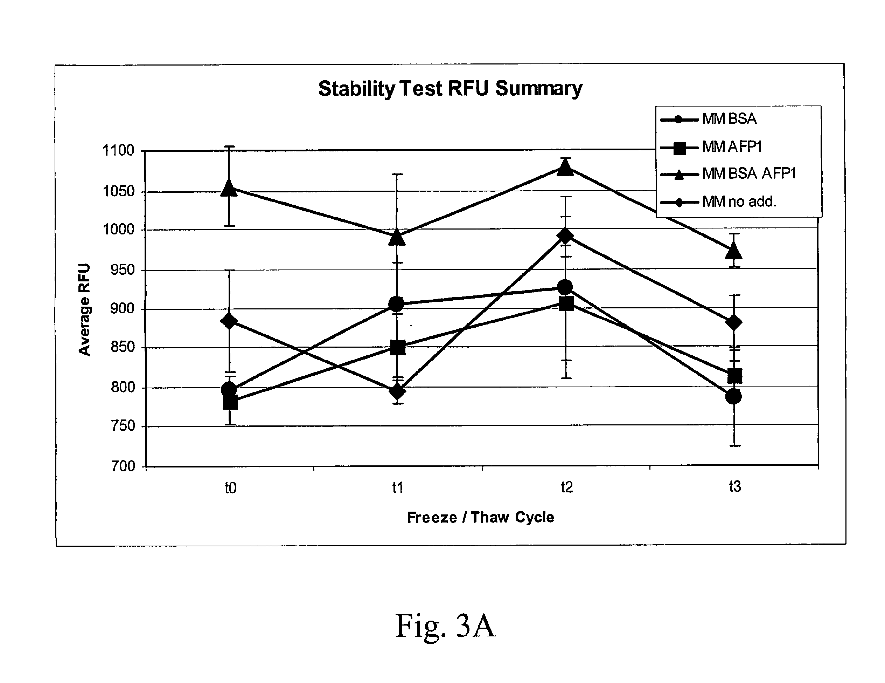

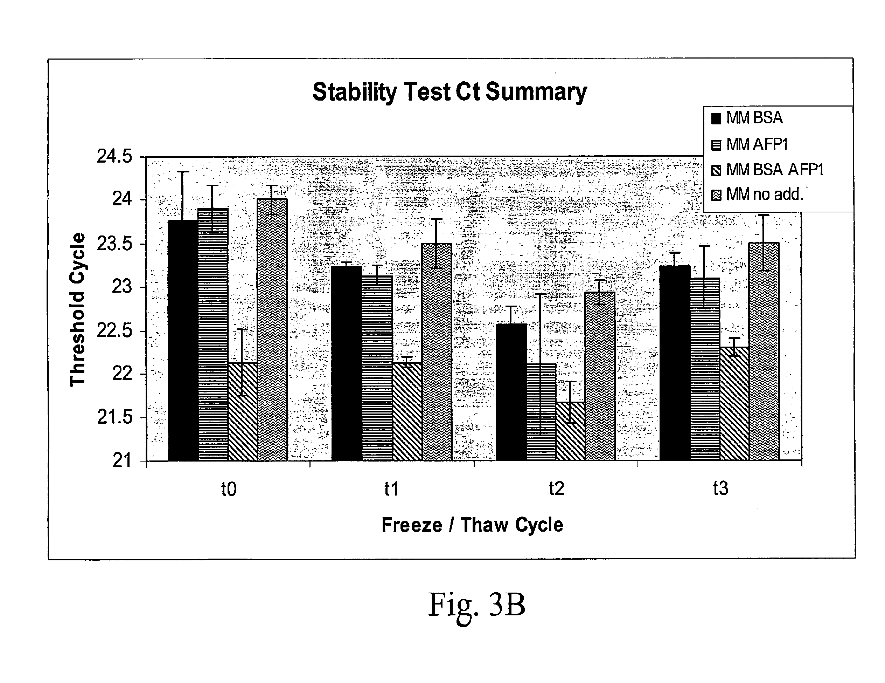

AFP1 :BSA containing real-time PCR samples were next tested for stability during freeze/thaw conditions over zero, one week , two weeks and three weeks at -20°C (see Figures 3A and 3B). Real-time PCR conditions were as described above except for the inclusion of the different amounts of carrier protein and AFP1. As shown in Figure 3A and 3B, the enhanced signal obtained during Real-Time PCR was maintained when AFP1 and BSA were added to the reaction mix (A) and the threshold cycle was also maintained as compared to the changes noted in BSA alone, AFP1 alone or no additives (B).

Similar Real Time PCR studies were performed using AFGP in the presence and absence of a carrier protein to determine whether the AFP1 effects were limited to AFP1 or were extendable to other AFP family members, and particularly to AFP family members having similar primary and secondary protein structure. AFGP includes a primary and secondary protein structure similar to AFP1 - particularly, strong similarity exists between the alanine-rich motif of AFP1 having an alpha helical amphiphilic secondary structure (see Table 1) and the alanine-alanine-threonine repeat disaccharide of AFGP. Like AFP1, AFGP in combination with BSA, had a significant effect on maintaining or lowering threshold cycle number as compared to control samples. Experimental parameters are shown in Table 3 below.

**Table 3: AFP1 and AFGP Reaction Set-up**

| **Buffer Master Mix for PCR (The combined master mixes are 2.5X)** | | | | | |
|---|---|---|---|---|---|
| Component | Stock | Final | µL/20µL rxn | MasterMix Volume | nuclease free water |
| 10X PCR buffer (1M sorbitol) | 10 | 2.5 | 5 | 800 | ---- |
| dNTP with UTP mix (mM) | 10 | 1 | 2 | 320 | ---- |
| glycerol (%) | 100 | 1 | 0.2 | 32 | ---- |
| DNA poly (U/µL) (Eppendorf Hot Master) | 5 | 2 | 0.4 | 64 | ---- |
| Total | | | 7.6 | 1216 | |
| Aliquot Master Mix into 4 tubes 167.2µL (22 rxns) each. Add 272.8 µL corresponding additive mix. | | | | | |

| **Buffer Master Mix With BSA** | | | | | |
|---|---|---|---|---|---|
| BSA (mg/ml) | 50 | 0.5 | 0.2 | 4.4 | 268.4 |

| **Buffer Master Mix With AFGP** | | | | | |
|---|---|---|---|---|---|
| AFGP (mg/ml) | 1 | 0.125 | 2.5 | 55 | 217.8 |

| **Buffer Master Mix With BSA and AFP1** | | | | | |
|---|---|---|---|---|---|
| BSA (mg/ml) | 50 | 0.5 | 0.2 | 4.4 | 213.4 |
| AFP1 (mg/ml) | 1 | 0.125 | 2.5 | 55 | ---- |

| **Buffer Master Mix No Additives** | | | | | |
|---|---|---|---|---|---|
| No Additive Control | 0 | 0 | 0 | 0 | 272.8 |

| **Buffer Master Mix With BSA and AFGP** | | | | | |
|---|---|---|---|---|---|
| BSA (mg/ml) | 50 | 0.5 | 0.2 | 4.4 | 213.4 |
| AFGP (mg/ml) | 1 | 0.125 | 2.5 | 55 | ---- |

| **Buffer Master Mix With BSA, AFP1, AFGP** | | | | | |
|---|---|---|---|---|---|
| BSA (mg/ml) | 50 | 0.5 | 0.2 | 4.4 | 158.4 |
| AFGP (mg/ml) | 1 | 0.125 | 2.5 | 55 | ---- |
| AFP1 (mg/ml) | 1 | 0.125 | 2.5 | 55 | ---- |

| **Buffer Master Mix With AFP1, AFGP** | | | | | |
|---|---|---|---|---|---|
| AFP1 (mg/ml) | 1 | 0.125 | 2.5 | 55 | ---- |
| AFGP (mg/ml) | 1 | 0.125 | 2.5 | 55 | ---- |

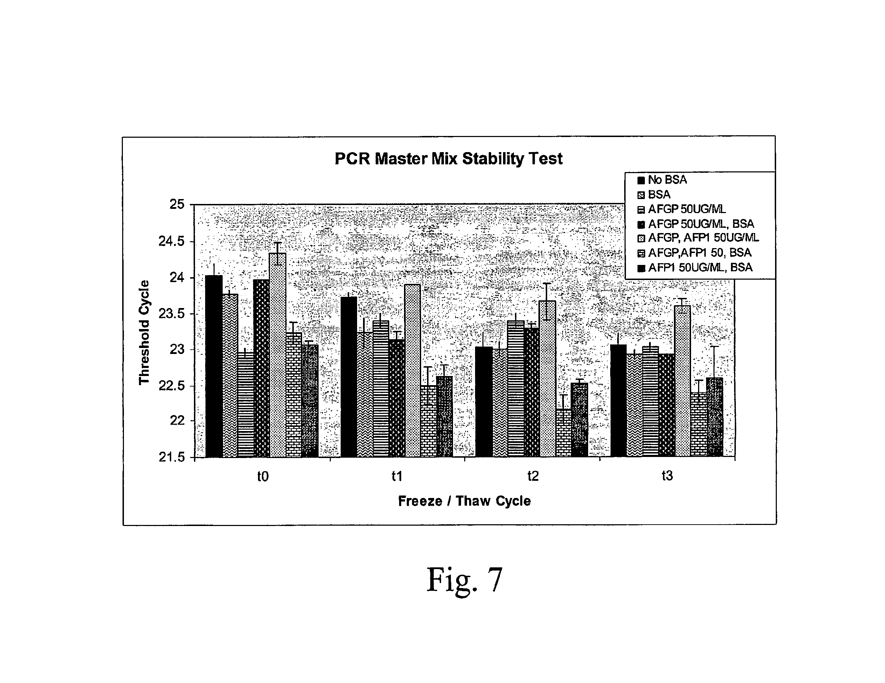

As shown in Figure 7, AFGP showed similar results in threshold cycle for fresh and freeze/thawed samples (-20°C) for one, two or three weeks. Note that the combination of AFP type I, AFGP and BSA provided the best overall performance during real time-PCR stability testing, as compared to the controls but also as compared to AFP type I alone, AFGP alone, AFP type I with BSA and AFGP with BSA.

The results in this Example show the utility of including AFP1 and AFGP, in the presence of a carrier protein, in Real-Time PCR reactions for facilitating the stability of the buffer during multiple freeze/thaw events at -20°C. This Example also provides data that at least two anti-freeze protein family members supported long term stability of a Real Time-PCR buffer, AFP type I and AFGP. The data provides strong evidence that proteins that exhibit similar anti-freeze protein like functional properties will be effective in similar manners to these proteins, and that the functional motif of AFP type I and AFGP (see Table 1 above) will be effective in a similar manner.

### Example 2: pH modification and Buffer Type Have Significant Effect on Optimizing Sorbitol, AFP1 Containing Real-Time PCR Buffer

Several real time-PCR buffer combinations were tested for signal amplification and for threshold cycle in the presence of a standard amount of both sorbitol and AFP1/BSA. Both the buffering component and salt were modified to provide different ionic concentrations and pHs. In particular, 25mM HEPES-KOH with 15mM KCL pH 8.0; 25mM TAPS-Tris with 15mM K GLUTpH 8.0; 25mM HEPES-Tris with 50 mM KCL pH 8.0; 25mM Bicine-Tris with 15mM K GLUT pH 8.7; 25mM HEPES-KOH with 15mM K GLUTpH 8.0; 25mM Bicine-Tris with 50mM K GLUT pH 8.0; 25mM TAPS-KOH with 15 mM KCL pH 8.0; 25mM Bicine-Tris with 15mM KCL pH 8.0; 25mM TAPS-Tris with 50mM KCL pH 8.0; and 25mM Bicine-Tris with 50mM KCI pH 8.4 were compared for ability to support highly accurate real time-PCR (each buffer also included 50 µg/ml AFP1, 200 µg/ml BSA and 100 mM sorbitol).

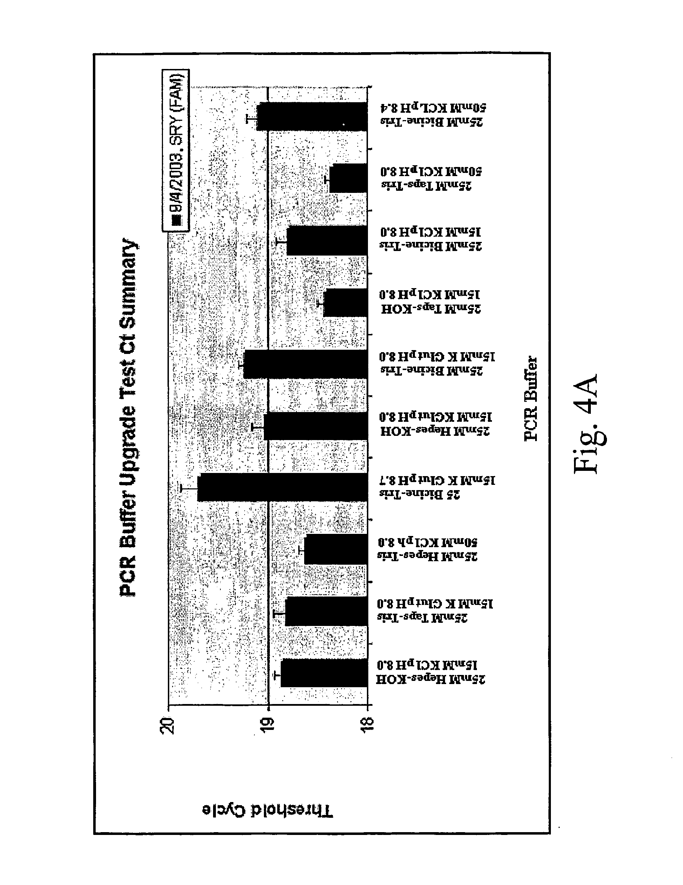

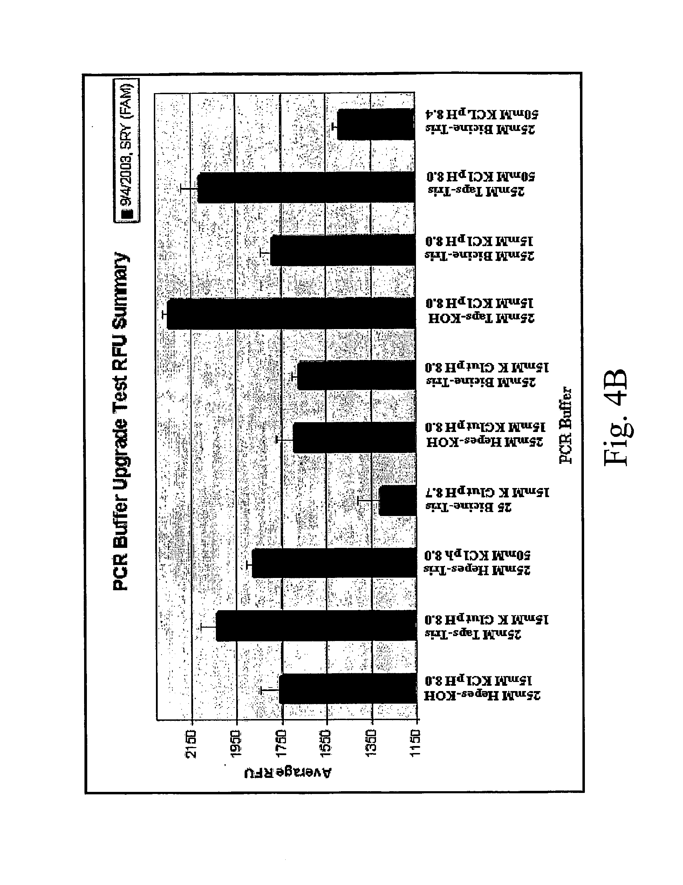

As shown graphically in Figure 4A and 4B (and Table 4), buffers composed on TAPS-KOH 150mM KCI pH 8.0 and TAPS-Tris 500mM KCI pH 8.0 provided the lowest threshold cycle and highest signal amplification in Real-Time PCR.

**Table 4: Real-Time PCR Buffer Test Data For Alternative Buffer Constituents**

| Buffer Type | Ave Ct | Standard Deviation of Ct | Ave RFU | Standard deviation of RFU |
|---|---|---|---|---|
| 25mM HEPES-KOH with15mM KCL pH 8.0 | 18.86667 | 0.057735 | 1751.46 | 90.14655 |
| 25mMTAPS-Tris with 15mM K GLUT pH 8.0 | 18.83333 | 0.11547 | 2037.563 | 64.23551 |
| 25mM HEPES-Tris with 50mM KCL pH 8.0 | 18.63333 | 0.057735 | 1873.903 | 32.29925 |
| 25mM Bicine-Tris with 15mM K GLUT, pH 8.7 | 19.7 | 0.173205 | 1308.253 | 99.58234 |
| 25mM HEPES-KOH with 15 mM K GLUTpH8 | 19.0333 | 0.11547 | 1691.167 | 78.98864 |
| 25mM Bicine-Tris with 15mM K GLUT pH 8.0 | 19.2333 | 0.057735 | 1666.757 | 31.04646 |
| 25mM TAPS-KOH with 15mM KCL pH 8.0 | 18.4333 | 0.057735 | 2243.12 | 29.99653 |
| 25mM Bicine-Tris with 15mM KCL, pH 8.0 | 18.8 | 0.1 | 1786.747 | 53.66013 |
| 25mM TAPS-Tris with 50mM KCL pH 8.0 | 18.36667 | 0.057735 | 2110.447 | 77.15043 |
| 25mM Bicine-Tris with 50mM KCI pH 8.4 | 19.1 | 0.1 | 1489.81 | 28.70814 |

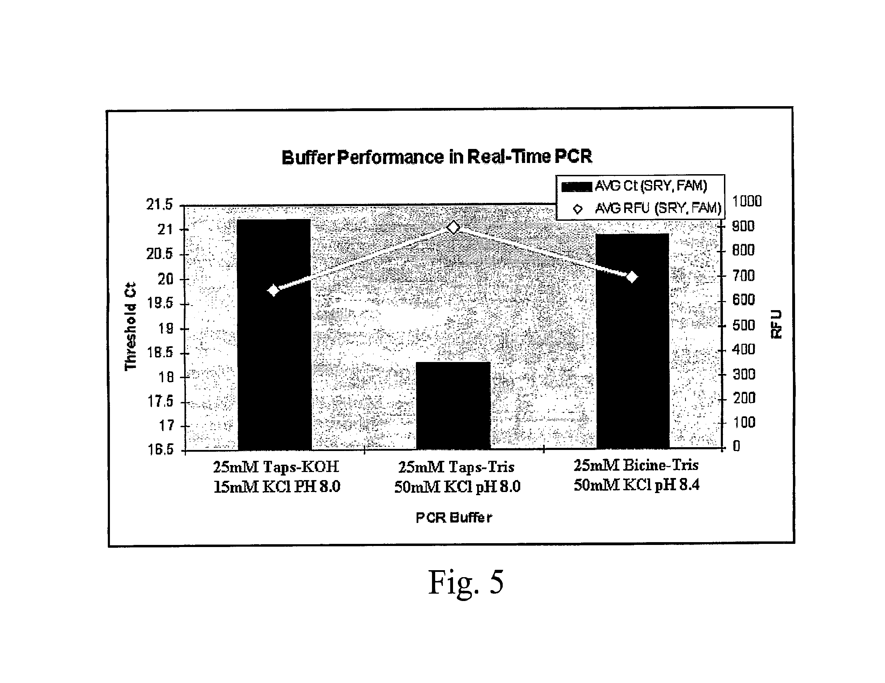

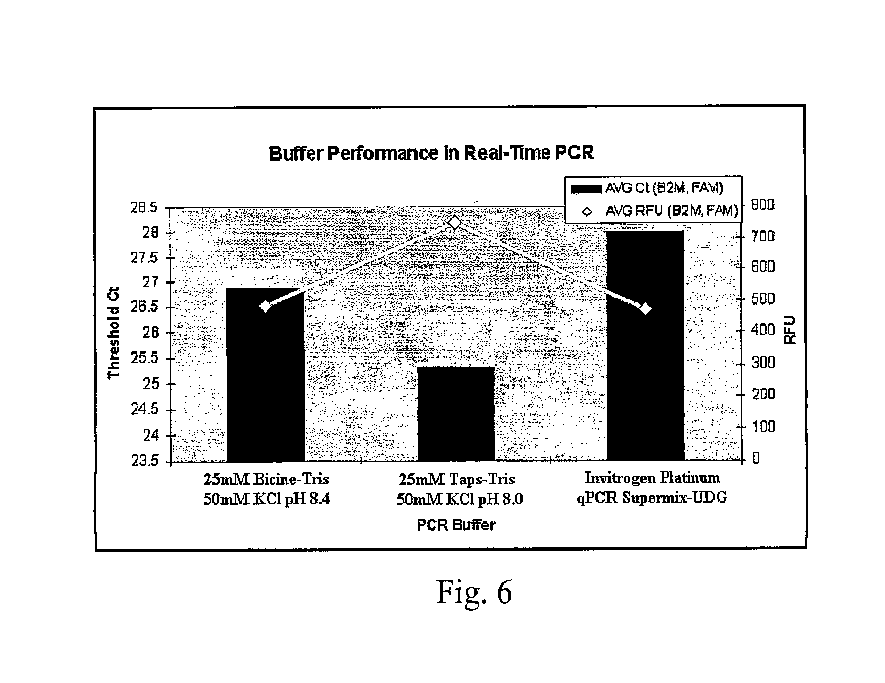

Further characterization was made on the buffer compositions by comparing the 25mM TAPS-KOH with 15mM KCLpH 8.0 , 25mM TAPS-Tris with 50mm KCLpH8.0 and 25mM Bicine-Tris with 50mM KCI pH 8.4 for ability to support Real-Time PCR. Results shown in Figure 5 graphically illustrate RFU and threshold cycle for each buffer condition, showing that 25mM TAPS-Tris with 50mM KClpH 8.0 out-performed both the 25mM TAPS-KOH with 15mM pH 8.0 and 25mM Bicine-Tris with 50mM KCI pH 8.4. Finally, results shown in Figure 6 illustrate that 25mM TAPS-Tris with 50 mM KCLpH 8.0 outperformed both 25mM Bicine-Tris with 50mM KCI pH 8.4, and Invitrogen Platinum qPCR Supermix-UDG (Invitrogen product number 11730-017). Real-Time PCR conditions for the Invitrogen Platinum qPCR Supermix-UDG were performed using manufacturer recommendation.

This data shows that a Real-Time PCR buffer composed of 25mM TAPS-Tris with 50mM KCI, pH of about 8, and including sorbitol, AFP1 and BSA, provided significant improvement in both signal size and threshold cycle over the Invitrogen based product. These same buffers also help stabilize buffer storage at -20°C for longer-term storage needs of the investigator.

### Example 3: AFP Containing PCR Master Mix Functionally Outperforms Competitor PCR Mix

An embodiment of a PCR Master Mix prepared in accordance with the present invention was functionally compared to Invitrogen Platinum qPCR Supermix-UDG for its ability to support long term stability within real time-PCR. Long term stability test experimental parameters are shown in Table 5. Cycling parameters included: 95°C for one minute and forty cycles of 95°C for twenty seconds, 56°C for ten seconds and 68°C for thirty seconds.

**Table 5: Long Term Stability Test**

| Aliquot 70µl of each of the RealMaster, 2.5X master mix into a biopure tube | | | | |
|---|---|---|---|---|
| Component | Stock | Final | µl/50µl rxn | 3.5 |
| 2.5X Real Master Mix +/- ROX(x) | 2.5 | 1 | 20 | 70 |

| **Primer-Probe Template Mix For 2.5X MM** | | | | |
|---|---|---|---|---|
| B2M fwd primer (µM) | 10 | 0.2 | 1 | 22 |
| B2M rev primer (µM) | 10 | 0.2 | 1 | 22 |
| B2M FAM probe (µM) | 7.5 | 0.15 | 1 | 22 |
| gDNA male (50ng/µl) | 50 | 1 | 1 | 22 |
| MBGW | | | 26 | 572 |
| Total | | | 30 | 660 |
| Add 105µl (3.5 rxn) of primer-probe-template mix to each aliquot of 2.5X master mix. Aliquot 87.5µl of each of the competitors 2X master mixes into a biopure tube. | | | | |
| 2X Invitrogen PCR probe Mix | 2 | 1 | 25 | 87.µ5 |

| **Primer-Probe-Template Mix For 2X MM** | | | | |
|---|---|---|---|---|
| B2M fwd primer (µM) | 10 | 0.2 | 1 | 33 |
| B2M rev primer (µM) | 10 | 0.2 | 1 | 33 |
| B2M FAM probe (µM) | 7.5 | 0.15 | 1 | 33 |
| gDNA male (50ng/µl) | 50 | 1 | 1 | 33 |
| MBGW | | | 21 | 693 |
| Total | | | 25 | 825 |
| Add 87.5µl (3.5 rxns) of primer-probe-template mix to each aliquot of 2X master mix | | | | |

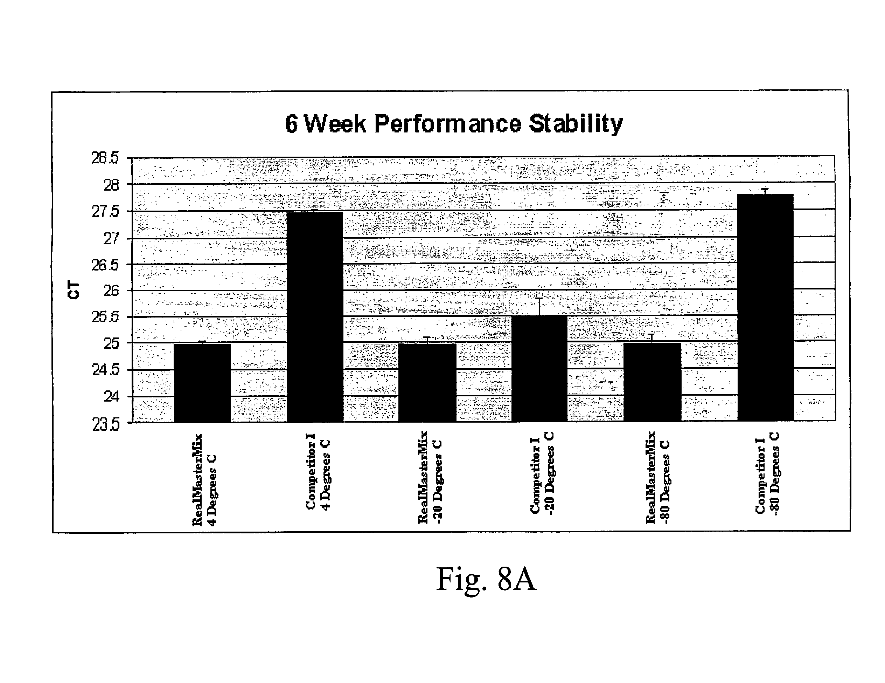

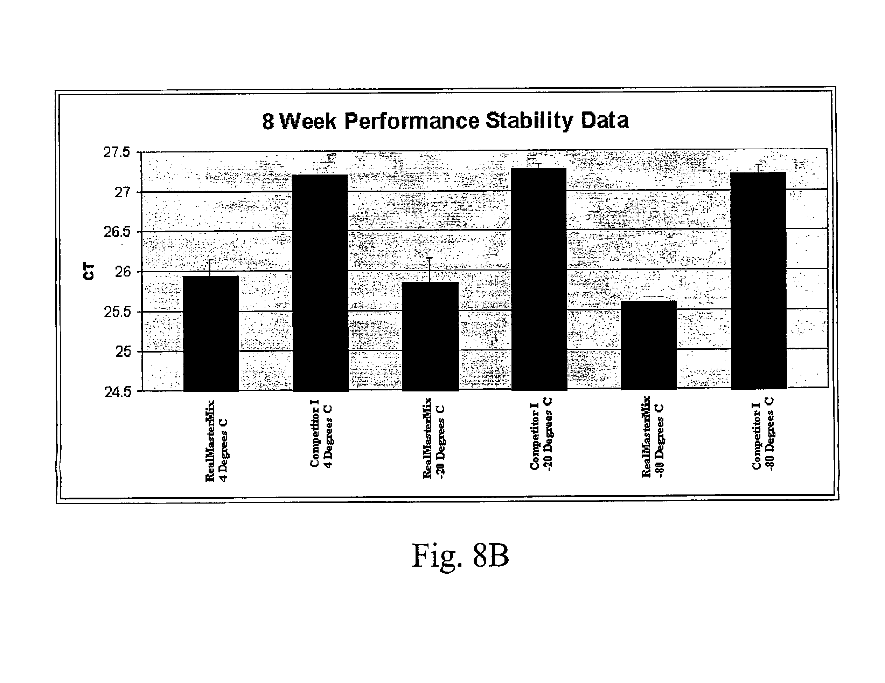

As shown in Figures 8A and 8B, the AFP containing Real Time-PCR buffer of the present invention supported stronger signal amplification (RFU)(8A) and earlier Ct(8B) at -4°C, -20°C and - 80°C as compared to Invitrogen Platinum qPCR Supermix-UDG over a six week course of experiments. The data illustrates that the buffers of the present invention provide comparable or better results for long-term stability, comparable to buffers sold as state of the art. As such, embodiments of the present invention have utility in providing long term buffer stability useful in real time PCR. Note that Invitrogen product was purchased and used per the manufacturer's recommendations.

### Example 4: Sorbitol Enhances Stability of Real Time-PCR Master Mix During Extended Periods of Storage at -20°

Sorbitol was included in real time-PCR master mix to determine its effects on storage stability of real time-PCR master mix buffers. Master mix samples were prepared with and without 100mM sorbitol, stored at 4, -20 or -80° C for one, two or three weeks and tested for overall performance in real time-PCR.

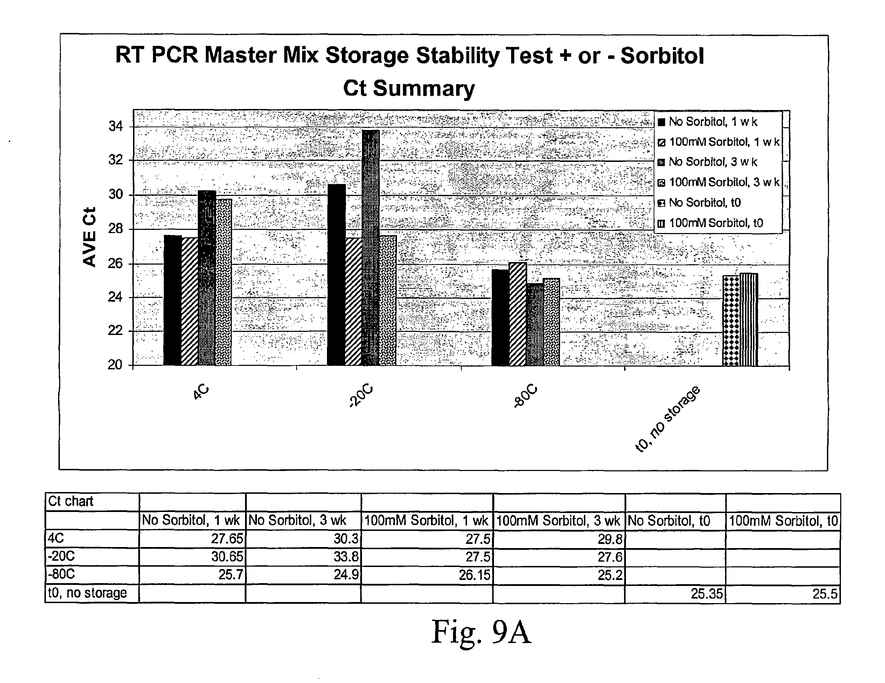

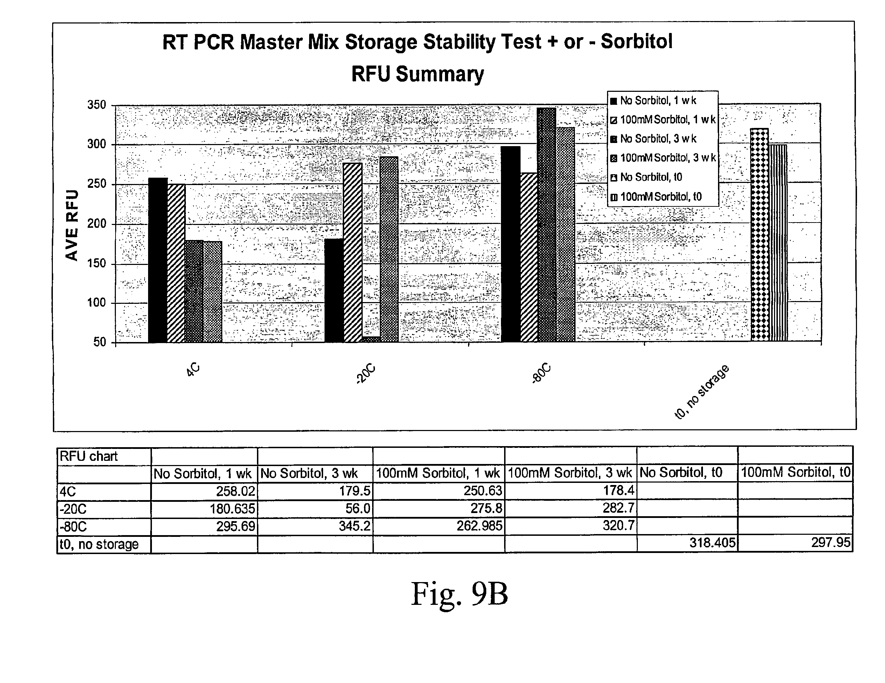

As shown in Figures 9A and 9B, 100mM sorbitol provided for an earlier Ct for reactions conducted with master mix buffers frozen at -20°C for one, two or three weeks (10A). Corresponding RFU values were also maintained in the sorbitol containing master mixes at this temperature. Samples stored at either 4°C or -80°C showed smaller or no change between the sorbitol containing and non-containing samples. It is believed that sorbitol is most effective at stabilizing master mix storage at -20°C due to the slow freeze nature of this temperature range. In comparison, storage of the master mix at either 4°C or -80°C was not expected to provide the same protection, as 4°C does not freeze samples and -80°C is a very fast snap freeze, less likely to involve formation of damaging water crystals, although as shown below some benefit is obtained.

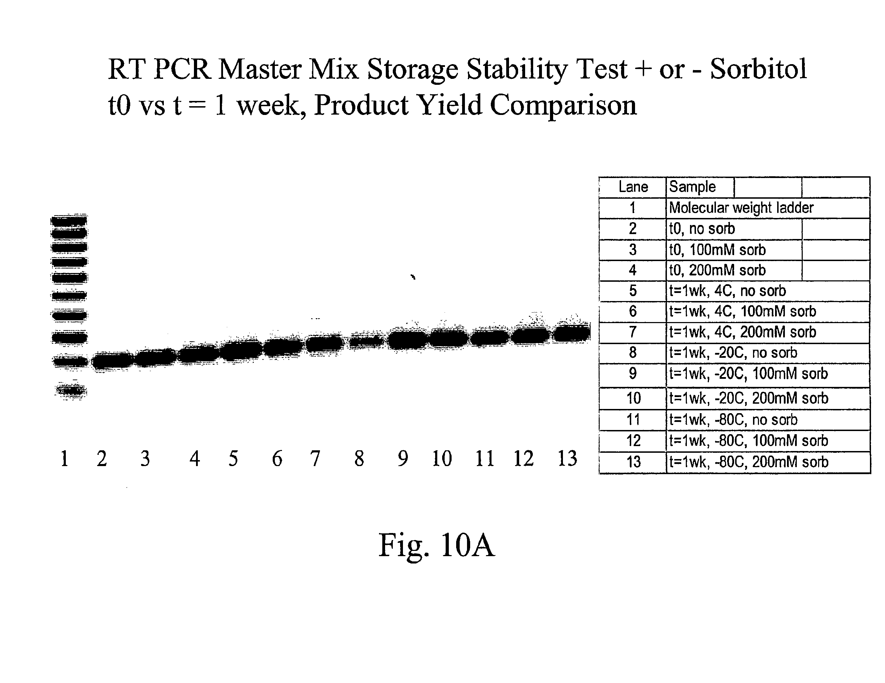

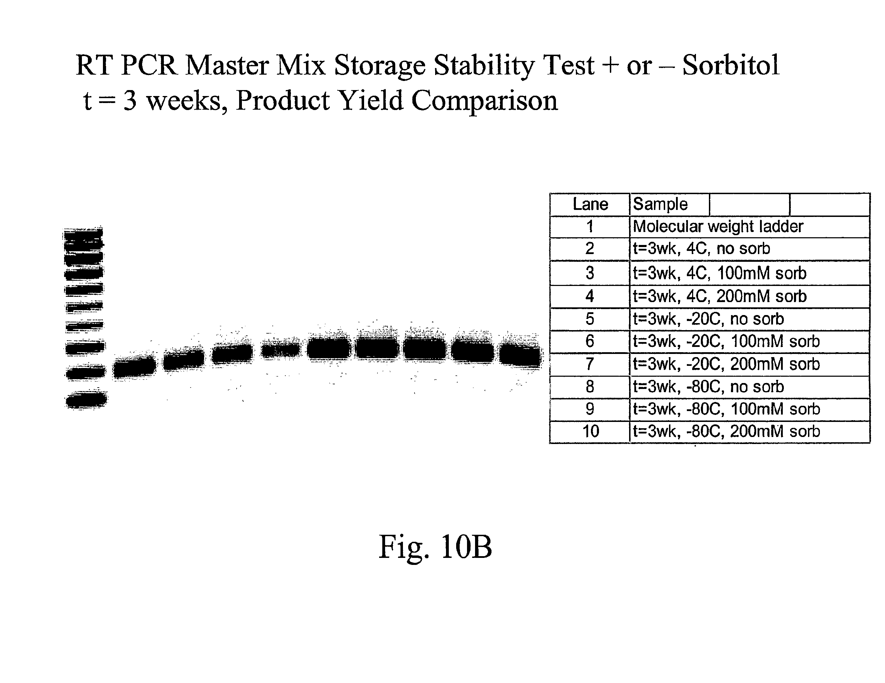

Figures 10A and 10B provide corresponding yield comparisons for one and three week storage, again indicating the usefulness of sorbitol in stabilizing the master mix at -20°C in RT-PCR. Note that the yield is significantly lowered when no sorbitol is included in the master mix stored at - 20°C (lane 8 in 10A and lane 5 in 10B). However, a dramatic increase for corresponding samples occurs when 100mM sorbitol is included in the master mix buffers.

### Example 5: AFP1 Provides Significant Stability Protection Over the Course of Numerous Freeze/Thaw Events

The effects of AFP1 on DNA poly based gDNA amplification was tested over the course of 15 freeze thaw cycles. Reaction mixes including a 2.5X RealMasterMix with or without ROX (5-carboxyx-rhodamine; triethyl ammonium salt). RealMasterMix is composed of 10X RealMaster Probe Buffer (0.5 mg/ml AFP1, 250 mm Taps, 103mm Tris, 500mm KC1, 50mm MgAc and 2mg/ml BSA), 10mm dNTP mix (Eppendorf product #W40460) and 100 Uµl HotMaster Taq DWA polymerase. Each reaction, therefore, includes approximately 200 µg/ml BSA and 50 µg/ml AFP1. Reactions were set up as shown in tables 6-7 and 8, using either a AB1 7000 thermal cycler or a Bio-Rad iCycler. Note that each reaction mix was gently vortexed.

**TABLE 6:**

| **REACTION COCKTAIL: ABI 7000 SDS-EPPENDORF Real MasterMix Probe ROS** | | | | |
|---|---|---|---|---|
| | **[STOCK]** | **[FINAL]** | **ul/50uL RXN** | **#RXNS 6.500** |
| 2.5X RealMasterMix + ROX (X) | 2.50 | 1.000 | 20.000 | 130.000 |
| B2M fwd primer (uM) | 10.00 | 0.200 | 1.000 | 6.500 |
| B2M rev primer (uM) | 10.00 | 0.200 | 1.000 | 6.500 |
| B2M FAM probe (uM) | 7.50 | 0.150 | 1.000 | 6.500 |
| MBGW | | | 26.000 | 169.000 |
| **TOTAL** | | | **49.000** | **318.500** |
| | | | | **4.500** |
| gDNA (ng/ul) | 50.00 | 1.000 | 1.000 | 4.500 |
| **TOTAL** | | | | **225.000** |
| | | | | |

| **REACTION COCKTAIL: BIO-RAD IQ iCYCLER--EPPENDORF RealMasterMix Probe** | | | | |
|---|---|---|---|---|
| | | | | |
| 2.5x RealMasterMix (X) | 2.5 | 1.000 | 20.000 | 130.000 |
| B2M fwd primer (uM) | 10.00 | 0.200 | 1.000 | 6.500 |
| B2M rev primer (uM) | 10.00 | 0.200 | 1.000 | 6.500 |
| B2MFAM probe (uM) | 7.50 | 0.150 | 1.000 | 6.500 |
| MBGW | | | 26.000 | 169.000 |
| **TOTAL** | | | **49.000** | **318.500** |
| | | | | **4.500** |
| gDNA (ng/ul) | 50.00 | 1.000 | 1.000 | 4.500 |
| **TOTAL** | | | | **225.000** |

**TABLE 7:**

| **THERMAL PROTOCOL: ABI 7000** | | | |
|---|---|---|---|
| | | | |

| | **# CYCLES** | **TEMPERATURE** | **TIME** |
|---|---|---|---|
| INITIAL DENATURATION | 1 | 95°C | 1:00* |
| CYCLING PARAMETERS | 40 | | |
| DENATURE | | 95°C | 00:20 |
| ANNEAL | | 56°C | 00:10 |
| EXTEND | | 68°C | 00:30 |
| HOLD | 1 | 4°C | Indefinite |
| *Incubate AB rxns 9:00 at 95oC then transfer all reactions to the 7000 | | | |

**TABLE 8:**

| **THERMAL PROTOCOL: BIO-RAD iCYCLER** | | | |
|---|---|---|---|
| | | | |

| | **# CYCLES** | **TEMPERATURE** | **TIME** |
|---|---|---|---|
| INITIAL DENATURATION | 1 | 95°C | 01:30 |
| CYCLING PARAMETERS | 40 | | |
| DENATURE | | 95°C | 00:20 |
| ANNEAL | | 56°C | 00:10 |
| EXTEND | | 68°C | 00.30 |
| HOLD | 1 | 4°C | Indefinite |

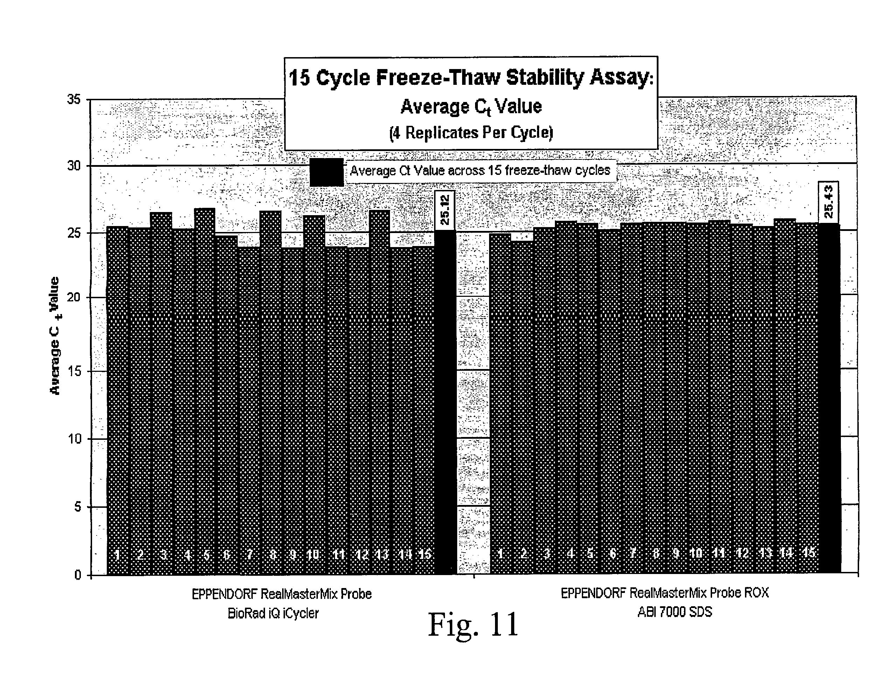

As shown in Fig 11, AFP1, mixed with BSA, provided protection to the DNA polymerase activity over the course of 15 freeze/thaw events. Each freeze/thaw cycle included a slow freeze to - 20°C and a thaw up to room temperature.

Each tested composition was evaluated for consistency of performance after each of the 15 freeze/thaw cycles under two alternative PCR methods. The data illustrates that AFP1, combined with BSA, functions to maintain DNA polymerase activity over the course of numerous freeze/thaw events. It is envisioned that this protection can be extended to other enzyme systems as well as for use in protecting enzymes over long periods of time at a freezing temperature.

The invention has been described with reference to specific examples. These examples are not meant to limit the invention in any way. It is understood for purposes of this disclosure, that various changes and modifications may be made to the invention that are well within the scope of the invention.

## Claims

1. An enzyme solution comprising an anti-freeze protein and an enzyme; wherein said enzyme is a polymerase and retains enzymatic activity after at least one freeze/thaw event.

2. The enzyme solution according to Claim 1, wherein said enzyme retains activity after more than ten freeze/thaw events.

3. The enzyme solution according to Claim 1 or 2, further comprising a buffer.

4. The enzyme solution according to Claim 3, wherein said buffer is zwitterionic.

5. The enzyme solution according to anyone of Claims 1 to 4, further comprising a carrier protein.

6. The enzyme solution according to Claim 5, wherein said carrier protein is bovine serum albumin (BSA).

7. The enzyme solution according to anyone of Claims 1 to 6, wherein said anti-freeze protein comprises an alanine-rich motif.

8. The enzyme solution according to anyone of Claims 1 to 7, wherein said anti-freeze protein is an AFP Type I protein.

9. The enzyme solution according to anyone of Claims 1 to 9, wherein the pH of the composition is from about 7.9 to about 8.9.

10. The enzyme solution according to anyone of Claims 1 to 9, further comprising a polyol.

11. The enzyme solution according to Claim 10, wherein the polyol is sorbitol, trehalose or mixtures thereof.

12. The enzyme solution according to anyone of Claims 1 to 11, wherein the anti-freeze protein has a concentration of from about 10 µg/ml to about 200 µg/ml.

13. The enzyme solution according to anyone of Claims 1 to 12, wherein said enzyme is a DNA polymerase and the addition of said enzyme solution to an amplification reaction mixture improves the sensitivity and yield of the nucleic acid amplification reaction.

14. A reaction mixture for use in a nucleic acid amplification reaction, comprising dNTPs and an enzyme solution according to Claim 13.

15. A method for enhancing the stability of an enzyme over the course of two or more freeze/thaw events, comprising the addition of an anti-freeze protein to an enzyme solution containing said enzyme prior to said freeze thaw events, wherein said enzyme is a polymerase.

16. A method for increasing the sensitivity and yield of a nucleic acid amplification reaction, comprising combining a target nucleic acid sequence with at least one primer in a reaction mixture according to Claim 14 and amplifying said target nucleic acid sequence, wherein the inclusion of said anti-freeze protein increases amplicon yield and sensitivity.

17. An improved method for detecting a target nucleic acid sequence in a sample or quantifying said target nucleic acid sequence ,
comprising combining said sample with at least one primer in a reaction mixture according to Claim 14 and amplifying said target nucleic acid sequence; wherein the inclusion of said anti-freeze protein increases signal intensity and improves the signal-to-noise ratio.

18. A kit comprising:
a solution comprising an anti-freeze protein and an enzyme, wherein the enzyme is a polymerase.

19. The kit of claim 18 wherein the solution further comprises a carrier protein.

## Patentansprüche

1. Eine Enzym-Lösung umfassend ein Anti-Gefrier-Protein und ein Enzym; wobei dieses Enzym eine Polymerase ist und enzymatische Aktivität nach mindestens einem Gefrier/Auftau-Ereignis behält.

2. Die Enzym-Lösung gemäß Anspruch 1, wobei das Enzym die Aktivität nach mehr als zehn Gefrier/Auftau-Ereignissen behält.

3. Die Enzym-Lösung gemäß Anspruch 1 oder 2, weiter umfassend einen Puffer.

4. Die Enzym-Lösung gemäß Anspruch 3, wobei dieser Puffer zwitterionisch ist.

5. Die Enzym-Lösung gemäß einem der Ansprüche 1 bis 4, weiter umfassend ein Träger-Protein.

6. Die Enzym-Lösung gemäß Anspruch 5, wobei dieses Träger-Protein Bovines Serumalbumin (BSA) ist.

7. Die Enzym-Lösung gemäß einem der Ansprüche 1 bis 6, wobei das Anti-Gefrier-Protein ein Alanin-reiches Motiv enthält.

8. Die Enzym-Lösung gemäß einem der Ansprüche 1 bis 7, wobei das Anti-Gefrier-Protein ein AFP Typ I Protein ist.

9. Die Enzym-Lösung gemäß einem der Ansprüche 1 bis 9, wobei der pH-Wert der Zusammensetzung zwischen etwa 7.9 bis etwa 8.9 liegt.

10. Die Enzym-Lösung gemäß einem der Ansprüche 1 bis 9, weiter umfassend ein Polyol.

11. Die Enzym-Lösung gemäß Anspruch 10, wobei das Polyol Sorbitol, Trehalose oder eine Mischung davon ist.

12. Die Enzym-Lösung gemäß einem der Ansprüche 1 bis 11, wobei das Anti-Gefrier-Protein eine Konzentration von etwa 10 µg/ml bis etwa 200 µg/ml aufweist.

13. Die Enzym-Lösung gemäß einem der Ansprüche 1 bis 12, wobei das Enzym eine DNA-Polymerase ist und das Hinzufügen der Enzym-Lösung zu einer Amplifikationsreaktions-Mischung die Sensitivität und die Ausbeute der Nukleinsäure-Amplifikationsreaktion verbessert.

14. Eine Reaktionsmischung zur Verwendung in einer Nukleinsäure Amplifikationsreaktion, beinhaltend dNTPs und eine Enzym-Lösung gemäß Anspruch 13.

15. Ein Verfahren, um die Stabilität eines Enzyms über den Verlauf von zwei oder mehr Gefrier/Auftau-Ereignissen zu erhöhen, umfassend die Zugabe eines Anti-Gefrier-Proteins zu einer Enzym-Lösung, die dieses Enzym vor den Gefrier/Auftau-Ereignissen beinhaltet, wobei das Enzym eine Polymerase ist.

16. Ein Verfahren, um die Sensitivität und die Ausbeute eine Nukleinsäure Amplifikationsreaktion zu erhöhen, umfassend die Kombination einer Zielnukleinsäure-Sequenz mit mindestens einem Primer in einer Reaktionsmischung gemäß Anspruch 14 und die Amplifikation dieser Zielnukleinsäure-Sequenz, wobei das Einbeziehen des Anti-Gefrier-Proteins die Amplicon-Ausbeute und die Sensitivität erhöht.

17. Ein verbessertes Verfahren, um eine Zielnukleinsäure-Sequenz in einer Probe zu detektieren oder diese Zielnukleinsäure-Sequenz zu quantifizieren, umfassend die Kombination dieser Probe mit mindestens einem Primer in einer Reaktionsmischung gemäß Anspruch 14 und die Amplifikation dieser Zielnukleinsäure-Sequenz, wobei das Einbeziehen des Anti-Gefrier-Proteins die Signalintensität erhöht und das Signal-Rauschen-Verhältnis verbessert.

18. Ein Kit umfassend:
eine Lösung umfassend ein Anti-Gefrier-Protein und ein Enzym, wobei das Enzym eine Polymerase ist.

19. Der Kit gemäß Anspruch 18, wobei die Lösung außerdem ein Träger-Protein beinhaltet.

## Revendications

1. Solution enzymatique comprenant une protéine antigel et une enzyme, dans laquelle ladite enzyme est une polymérase et retient une activité enzymatique après au moins un épisode de gel/dégel.

2. Solution enzymatique selon la revendication 1, dans laquelle ladite enzyme retient une activité après plus de dix épisodes de gel/dégel.

3. Solution enzymatique selon l'une des revendications 1 et 2, qui comprend en outre un tampon.

4. Solution enzymatique selon la revendication 3, dans laquelle ledit tampon est zwitterionique.

5. Solution enzymatique selon l'une quelconque des revendications 1 à 4, qui comprend en outre une protéine porteuse.

6. Solution enzymatique selon la revendication 5, dans laquelle ladite protéine porteuse est de l'albumine de sérum bovin (BSA).

7. Solution enzymatique selon l'une quelconque des revendications 1 à 6, dans laquelle ladite protéine antigel comprend un motif riche en alanine.

8. Solution enzymatique selon l'une quelconque des revendications 1 à 7, dans laquelle ladite protéine antigel est une protéine AFP de type 1.

9. Solution enzymatique selon l'une quelconque des revendications 1 à 9, dans laquelle le pH de la composition varie d'environ 7,9 à environ 8,9.

10. Solution enzymatique selon l'une quelconque des revendications 1 à 9, qui comprend en outre un polyol.

11. Solution enzymatique selon la revendication 10, dans laquelle le polyol est du sorbitol, du tréhalose ou des mélanges de ceux-ci.

12. Solution enzymatique selon l'une quelconque des revendications 1 à 11, dans laquelle la concentration de la protéine antigel varie d'environ 10 µg/ml à environ 200 µg/ml.

13. Solution enzymatique selon l'une quelconque des revendications 1 à 12, dans laquelle ladite enzyme est une ADN polymérase et l'ajout de ladite solution enzymatique à un mélange réactionnel d'amplification améliore la sensibilité et le rendement de la réaction d'amplification d'acides nucléiques.

14. Mélange réactionnel pour utilisation dans une réaction d'amplification d'acides nucléiques, qui comprend des dNTP et une solution enzymatique selon la revendication 13.

15. Procédé pour améliorer la stabilité d'une enzyme sur une période de deux épisodes ou plus de gel/dégel, qui comprend l'ajout d'une protéine antigel à une solution enzymatique contenant ladite enzyme avant lesdits épisodes de gel, dans lequel ladite enzyme est une polymérase.

16. Procédé pour accroître la sensibilité et le rendement d'une réaction d'amplification d'acides nucléiques, qui comprend la combinaison d'une séquence cible d'acides nucléiques avec au moins une amorce dans un mélange réactionnel selon la revendication 14 et l'amplification de ladite séquence cible d'acides nucléiques, dans lequel l'inclusion de ladite protéine antigel augmente le rendement et la sensibilité de l'amplicon.

17. Procédé amélioré pour la détection d'une séquence cible d'acides nucléiques dans un échantillon ou la quantification de ladite séquence cible d'acides nucléiques, qui comprend la combinaison dudit échantillon avec au moins une amorce dans un mélange réactionnel selon la revendication 14 et l'amplification de ladite séquence cible d'acides nucléiques, dans lequel l'inclusion de ladite protéine antigel augmente l'intensité du signal et améliore le rapport signal sur bruit.

18. Trousse comprenant :
une solution comprenant une protéine antigel et une enzyme, dans laquelle l'enzyme est une polymérase.

19. Trousse selon la revendication 18, dans laquelle la solution comprend en outre une protéine porteuse.
